(19) Europäisches Patentamt
European Patent Office
Office européen des brevets

(11) EP 1 028 751 B1

(12) EUROPEAN PATENT SPECIFICATION

(45) Date of publication and mention of the grant of the patent:
**31.12.2008 Bulletin 2009/01**

(21) Application number: **98953633.9**

(22) Date of filing: **16.10.1998**

(51) Int Cl.:
***A61K 39/395** (2006.01)* ***C07K 16/00** (2006.01)*
***C12P 21/00** (2006.01)*

(86) International application number:
**PCT/US1998/021925**

(87) International publication number:
**WO 1999/022764 (14.05.1999 Gazette 1999/19)**

(54) **METHODS AND COMPOSITIONS COMPRISING GLYCOPROTEIN GLYCOFORMS**

METHODEN UND ZUSAMMENSETZUNGEN BESTEHEND AUS GLYKOPROTEIN-GLYKOFORMEN

COMPOSITIONS RENFERMANT DES GLYCOFORMES DE GLYCOPROTEINE ET METHODES AFFERENTES

(84) Designated Contracting States:
**AT BE CH CY DE DK ES FI FR GB GR IE IT LI LU MC NL PT SE**

(30) Priority: **31.10.1997 US 962497**

(43) Date of publication of application:
**23.08.2000 Bulletin 2000/34**

(73) Proprietor: **Genentech, Inc.**
**South San Francisco CA 94080-4990 (US)**

(72) Inventor: **RAJU, T., Shantha**
**San Mateo, CA 94402 (US)**

(74) Representative: **Kiddle, Simon John et al**
**Mewburn Ellis LLP**
**23 Kingsway**
**London WC2B 6HP (GB)**

(56) References cited:
**WO-A-98/58964**

- **KLAMA K ET AL: "The abnormalities in the glycosylation of IgG" REUMATOLOGIA, vol. 34, no. 1, 1996, pages 26-30, XP002081302**
- **LIFELY M R (REPRINT) ET AL: "Glycosylation and biological-activity of CAMPATH-1H expressed in different cell-lines and grown under different culture conditions" GLYCOBIOLOGY, (DEC 1995) VOL. 5, NO. 8, PP. 813-822. ISSN: 0959-6658., XP002096118**
- **WRIGHT A ET AL: "Effect of altered CH2 -associated carbohydrate structure on the functional properties and in vivo fate of chimeric mouse-human immunoglobulin G1." JOURNAL OF EXPERIMENTAL MEDICINE, (1994 SEP 1) 180 (3) 1087-96. JOURNAL CODE: I2V. ISSN: 0022-1007., XP000676306 United States**
- **BOYD P N ET AL: "The effect of the removal of sialic acid, galactose and total carbohydrate on the functional activity of CAMPATH-1H" MOLECULAR IMMUNOLOGY, vol. 32, no. 17/18, 1995, pages 1311-1318, XP000676340 cited in the application**
- **UMANA P ET AL: "A mathematical model of N - linked glycoform biosynthesis" BIOTECHNOLOGY AND BIOENGINEERING, (20 SEP 1997) VOL. 55, NO. 6, PP. 890-908. PUBLISHER: JOHN WILEY & SONS INC, 605 THIRD AVE, NEW YORK, NY 10158-0012. ISSN: 0006-3592., XP002096119**
- **DWEK R A ET AL: "Glycobiology: 'the function of sugar in the IgG molecule'." JOURNAL OF ANATOMY, (1995 OCT) 187 ( PT 2) 279-92. JOURNAL CODE: HBB. ISSN: 0021-8782., XP002096120 ENGLAND: United Kingdom**
- **WEITZHANDLER M ET AL: "Analysis of carbohydrates on IgG preparations." JOURNAL OF PHARMACEUTICAL SCIENCES, (1994 DEC) 83 (12) 1670-5. JOURNAL CODE: JO7. ISSN: 0022-3549., XP002096121 United States cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 028 751 B1

- JEFFERSIS ET AL.: "Glycosylation of antibody molecules: Structural and functional significance." CHEMICAL IMMUNOLOGY, vol. 65, 1997, pages 111-128, XP002096122 cited in the application
- WRIGHT ET AL.: "Effect of glycosylation on antibody funtion: implications for genetic engineering." TIBTECH, vol. 15, 1997, pages 26-32, XP004016809 cited in the application
- WORMALD ET AL.: "Variations in oligosaccharide-Protein interactions in immunoglobulin G determine the site-specific glycosylation profiles and modulate the dynamic motion of the Fc oligosaccharides." BIOCHEMISTRY, vol. 36, 1997, pages 1370-1380, XP002096123 cited in the application
- RAJU ET AL: "Biological significance and structural features of immunoglobulin glycoforms" BOOK OF ABSTRACTS. ACS NATIONAL MEETING, 29 April 1998, page COMPLETE 1 XP002081304

## Description

## Background of the Invention

### Field of the Invention

This invention relates to glycoprotein glycoforms as well as to novel compositions comprising the glycoforms of the invention. More particularly, the invention relates to glycoprotein compositions which comprise glycoproteins such as an immunoglobulin, antibody or immunoadhesin having an immunoglobulin CH2 domain containing particular N-linked glycans. The invention further relates to methods for producing, detecting, enriching and purifying the glycoprotein glycoforms. The invention further relates to pharmaceutical compositions, methods of using the compositions, as well as articles of manufacture comprising the compositions.

### Description of Related Disclosures

Differences in glycosylation patterns of recombinantly produced glycoproteins have recently been the topic of much attention in the scientific community as recombinant proteins produced as probable prophylactics and therapeutics approach the clinic. Antibodies or immunoglobulins (Ig) are glycoproteins that play a central role in the humoral immune response. Antibodies and antibody-like molecules such as immunoadhesins (U.S. Patent Nos. 5,116,964 and 5,565,335) have been prepared for clinical uses; for example, TNFR-IgG (Ashkenazi et al., (1991) Proc. Natl. Acad. Sci. USA 88: 1-535-1053. U.S. Patent No. 5,610,297 and "Ro45-2081 (TNFR55-IgG1) in the Treatment of Patients with Severe Sepsis and Septic Shock: Preliminary Results" Abraham et al., (1995) in Sec. Intern. Autumnal Them. Meeting on Sepsis, Deauville, France); anti-IL-8 (St John et al., (1993), Chest, 103:932 and International Publication No. WO 95/23865); anti-CD11a (Filcher et al., Blood, 77:249-256, Steppe et al., (1991), Transplant Intl. 4:3-7, and Hourmant et al., (1994), Transplantation 58:377-380); anti-IgE (Presta et al., (1993), J. Immunol. 151:2623-2632, and International Publication No. WO 95/19181); anti-HER2 (Carter et al., (1992), Proc. Natl. Acad. Sci. USA, 89:4285-4289, and International Publication No. WO 92/20798); anti-VEGF (Jin Kim et al., (1992) Growth Factors, 7:53-64, and International Publication No. WO 96/30046); and anti-CD20 (Maloney et al., (1994) Blood. 84:2457-2466, Liu et al., (1987) J. Immunol., 130: 3521-3526).

Antibodies are glycosylated at conserved positions in their constant regions (Jefferis and Lund ( 1997) Chem. Immunol. 65:111-128; Wright and Morrison (1997) TibTECH 15:26-32). The oligosaccharide side chains of the immunoglobulins affect the protein's function (Boyd et al., (1996) Mol. Immunol. 32:1311-1318; Wittwer A., and Howard, S.C. (1990) Biochem. 29:4175-4180) and the intramolecular interaction between portions of the glycoprotein which can affect the conformation and presented three-dimensional surface of the glycoprotein (Jefferis and Lund supra; Wyss and Wagner (1996) Curr. Opin. Biotech. 7:409-416; Hart, (1992) Curr. Opin. Cell Biol., 4:1017-1023; Goochee, et al., (1991) Bio/Technology, 9:1347-1355; Parekh, R.B., (1991) Curr. Opin. Struct. Biol., 1:750-754). Oligosaccharides may also serve to target a given glycoprotein to certain molecules based upon specific recognition structures. For example, it has been reported that in agalactosylated IgG, the oligosaccharide moiety 'flips' out of the inter-CH2 space and terminal N-acetylglucosamine residues become available to bind mannose binding protein (Malhotra et al., (1995) Nature Med. 1: 237-243). It has also been reported that the presence of nonreducing terminal galactose residues in antibody CH2 domains may be important for binding of IgG to Clq and Fc receptors (Tsuchiya et al., (1989) J. Rheum. 16:285-290).

CAMPATH-1H is a recombinant humanized murine monoclonal IgG1 antibody which recognizes the CDw52 antigen of human lymphocytes (Sheeley et al., (1997) Analytical Biochem. 247: 102-110). Removal by glycopeptidase-of the oligosaccharides from CAMPATH-1H produced in chinese hamster ovary (CHO) cells resulted in a complete reduction in complement-mediated cell lysis (CMCL) (Boyd et al., (1996) Mol. Immunol. 32:1311-1318); whereas selective removal of sialic acid residues using neuraminidase resulted in no loss of CMCL. CAMPATH-1H treated with β-galactosidase, which is expected to remove terminal galactosyl residues, was found to reduce CMCL by less than one-half (Boyd et al. supra).

Since the cell type used for expression of recombinant glycoproteins as potential human therapeutics is rarely the native cell, significant variations in the glycosylation pattern of the glycoproteins can be expected. Tissue plasminogen activator produced in different cell types results in heterogeneously glycosylated molecules (Parekh, et al., (1989) Biochemistry 28: 7644-7662). The same is true for immunoglobulins (Hse, T.A. et al., (1997) J. BioL Chem. 272:9062-9070).

Much attention has been paid to the factors which affect lycosylation during recombinant protein production such as growth mode (adherent or suspension), media formulation, culture density, oxygenation, pH, purification schemes and the like (Werner, R. and Noe, W. (1993), Drug Res. 43:1134-1249; Hayter et al., (1992) Biotech, and Bioeng. 39: 327-335; Borys et al., (1994) Biotech and Bioeng. 43:505-514; Borys et al., (1993) Bio/technology 11:720-724; Hearing et al., (1989) J. Cell Biol. 108:339-353; Goochee el al., in Frontiers in Bioprocessing II, Todd et al., eds (1992) American Chemical Society pp.199-240; U.S. Patent No. 5,096,816; Chotigeat, W., (1994) Cytotech. 15:217-221). Several groups

have investigated the process parameters that affect the production of recombinant proteins, especially the effect of media composition in the production of recombinant proteins (Park et al., (1992) Biotech. Bioeng. 40:686-696; Cox and McClure, (1983) In Vitro, 19:1-6; Mizutani et al., (1992) Biochem. Biophys. Res. Comm. 187:664-669; Le Gros et al., (1985) Lymph. Res. 4(3):221-227). Various methods have been proposed to alter the glycosylation pattern achieved in a particular host organism including introducing or overexpressing certain enzymes involved in oligosaccharide production (U.S. Patent No. 5,047,335 U.S. Patent No. 5,510,261). These schemes are not limited to intracellular methods (U.S. Patent No. 5,278,299).

KLAMA K ET AL: "The abnormalities in the glycosylation of IgG" REUMATOLOGIA, vol. 34, no. 1, 1996, pages 26-30, discusses glycosylation as the main post-translational modification of protein molecules. Human IgG lacking a galactose from the oligosaccharides on the CH2 domain correlate with disease severity in a number of autoimmune diseases. Agalactosyl IgG is connected with rheumatoid arthritis and may be involved in its immunopathogenesis.

While some work has been done to evaluate the structure of the N-linked glycans attached to the heavy chain of clinically relevant antibodies, these studies indicate that various host cells are capable of differential N-glycan processing. Analysis of the produced glycoprotein reveals heterogeneous glycoforms (Wormald et al., (1997) Biochemistry 36: 1370-1380). Glycosylation differences in antibodies are generally confined to the constant domain and may influence the antibodies' structure (Weitzhandler et al., (1994) J. Pharm. Sci. 83:1760). It is therefore important to ensure that the glycosylation pattern of glycoprotein products produced for clinical use is uniform throughout and between production lots but also that the favorable in vivo properties of the antibodies are at least retained.

## Summary of the Invention

The present invention provides for compositions as defined in the claims comprising glycoprotein agents substantially free of particular glycoforms of the glycoprotein agent. It is a feature of the present invention that when administered to animals including humans, pharmaceutical compositions comprising the novel compositions, in preferred embodiments, advantageously exhibit superior in vivo properties. Thus, the novel compositions may be used wherever the glycoprotein pharmaceutical agent is used and advantageously provide improved properties as well as increased uniformity between and throughout production lots. The preparations of the invention can be incorporated into solutions, unit dosage forms such as tablets and capsules for oral delivery, as well as into suspensions, ointments and the like, depending on the particular drug or medicament and its target area.

According to a particular aspect of the invention as defined in the claims compositions are provided comprising a glycoprotein having an immunoglobulin CH2 domain wherein the CH2 domain has at least one N-linked oligosaccharide and wherein substantially all of the oligosaccharide is a G-2 oligosaccharide as defined herein. Also provided are compositions as defined in the claims comprising a glycoprotein having at least one immunoglobulin CH2 domain wherein the N-linked oligosaccharides of the CH2 domain are substantially of the G2 and G-2 variety. The composition is substantially free of glycoproteins comprising an immunoglobulin CH2 domain wherein the N-linked oligosaccharide is a G1, G0 or G-1 oligosaccharide. In preferred aspects the glycoprotein is an antibody and especially a monoclonal antibody. The invention further provides for a method of producing the preparations of the invention.

The invention encompasses pharmaceutical compositions comprising the glycoform preparations of the invention. The compositions are preferably sterile. Where the composition is an aqueous solution, preferably the glycoprotein is soluble. Where the composition is a lyophilized powder, preferably the powder is reconstitutable in an appropriate solvent.

In still another aspect, the invention involves means for the treatment of a disease state by administering to a mammal in need thereof a therapeutically effective dose of the pharmaceutical compositions of the invention.

It is a further object of the invention to provide the glycoform preparations in an article of manufacture or kit that can be employed for purposes of treating a disease or disorder.

Brief Description of the Drawings

Figure 1 A, Figure 1B and Figure 1C depict oligosaccharide analysis of an anti-CD20 monoclonal antibody C2B8 by capillary electrophoresis with laser-induced fluorescence detection. C2B8 produced in 400L batch-fed culture produced at least four glycoforms of C2B8 (Figure 1A). Figure 1B depicts the same C2B8 preparation treated with β-galactosidase. Figure 1C depicts the preparation further treated with N-acetylβ-D-glucosaminosidase A single G-2 glycoform preparation was obtained (G-2).

Figure 2 depicts the binding of C2B8 to Clq using the procedure of Reff et al., (1994) Blood 83:435-445. Both G2 and G-2 preparations bound C1q to a greater extent than control samples exhibiting heterogeneous glycoforms.

Figure 3 depicts the bioactivity of the G2 and G-2 glycoform preparations compared with the preparations having

all carbohydrate removed (No) and preparations having only galactose removed (G0) in a rabbit/human complement lysis assay.

Figure 4A and Figure 4B depict oligosaccharide analysis of an anti-CD20 monoclonal antibody C2B8 by capillary electrophoresis with laser-induced fluorescence detection. C2B8 produced in 400L batch-fed culture produced at least three glycoforms of C2B8 (Figure 4A). Figure 4B depicts the same C2B8 preparation after treatment with β1-4 galactosyltransferase according to the present invention. A single G2 glycoform preparation was obtained.

Figure 5 depicts analysis of an anti-VEGF monoclonal antibody by capillary electrophoresis. It can be seen that anti-VEGF produced in CHO cell culture contained at least three glycoforms forming a heterogeneous composition. The same anti-VEGF after treatment with β-1-4 galactosyltransferase according to the present invention produced a single G2 glycoform.

Figure 6 depicts analysis of an anti-IgE monoclonal antibody by capillary electrophoresis. It can be seen that anti-IgE produced in CHO cell culture contained at least three glycoforms forming a heterogeneous oligosaccharide population. The same anti-IgE CHO cell composition after treatment with β-1-4 galactosyltransferase according to the present invention produced a single G2 glycoform.

Figure 7 depicts analysis of an anti-HER2 monoclonal antibody by capillary electrophoresis. It can be seen that anti-HER2 produced in CHO cell culture contained at least three glycoforms forming a heterogeneous oligosaccharide population. The same anti-HER2 CHO composition after treatment with β-1-4 galactosyltransferase according to the present invention produced a single G2 glycoform.

Figure 8 depicts a representative SDS polyacrylamide gel analysis of an anti-CD20 monoclonal antibody under non-reducing conditions. Lane 1 is molecular weight standards, Lane 2 is the G2 glycoform of C2B8; Lane 3 is the C2B8 preparation treated with galactosidase to remove galactose residues from the oligosaccharides; Lane 4 is the CHO derived C2B8 preparation treated with glycopeptidase-F for the removal of intact oligosaccharide; Lane 5 is the C2B8 antibody from CHO production; Lane 6 is CHO-derived C2B8 after incubation at 37 C for 24 hours; lane 7 is CHO-derived C2B8 and BSA. The representative gel shows that the molecular size of the C2B8 molecule remains intact after treatment with the galactosyltransferase. The G2 glycoform does not disrupt the primary structure of the antibody.

Figure 9 depicts the same material described above analyzed by polyacrylamide gel electrophoreses under reducing conditions. The C2B8 heavy and light chains remain intact.

Figure 10A and Figure 10B depict far and near UV circular dichroism (CD) spectra of C2B8 antibody from CHO culture and the G2 glycoform. As circular dichroism is sensitive to secondary structure (Provencer and Glockner (1981), Biochem. 20:33-37) it can be concluded that the G2 glycoform has the same secondary structure as C2B8 of heterogenous glycan composition.

Figure 11 depicts the bioactivity of the G2 glycoform preparation compared with the heterogeneous composition for C2B8 in a rabbit complement lysis assay.

Figure 12 depicts the correlation of bioactivity and galactose content in the G2 glycoform. The G2 glycoform preparation was at least 1.5 times more active in this assay than that produced under typical cell culture conditions.

## Detailed Description of the Preferred Embodiments

### Definitions

The carbohydrate moieties of the present invention will be described with reference to commonly used nomenclature for the description of oligosaccharides. A review of carbohydrate chemistry which uses this nomenclature is found in Hubbard and Ivatt (1981) Ann., Rev. Biochem. 50:555-583. This nomenclature includes, for instance, Man, which represents mannose; GlcNAc, which represents 2-N-acetylglucosamine; Gal which represents galactose; Fuc for fucose; and Glc, which represents glucose. Sialic acids are described by the shorthand notation NeuNAc, for 5-N-acetylneuraminic acid, and NeuNGc for 5-glycolylneuraminic acid (J. Biol. Chem, 1982 257:3347; J. Biol. Chem., 1982, 257:3352).

The "CH2" domain of the present invention is meant to describe an immunoglobulin heavy chain constant CH2 domain. In defining an immunoglobulin CH2 domain reference is made to immunoglobulins in general and in particular to the domain structure of immunoglobulins as applied to human IgG1 by Kabat E.A. (1978) Adv. Protein Chem. 32: 1-75. Accordingly, immunoglobulins are generally heterotetrameric glycoproteins of about 150,000 daltons, composed of two identical light (L) chains and two identical heavy (H) chains. Each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has an amino terminal variable domain (VH) followed by carboxy terminal constant domains. Each light chain has a variable N-terminal domain (VL) and a C terminal constant domain; the constant domain of the light chain is aligned with the first constant domain (CH1) of the heavy chain, and the light chain variable domain is aligned with the variable domain of the heavy chain. According to the domain definition of immunoglobulin polypeptide chains, light (L) chains have two

conformationally similar domains VL and CL; and heavy chains have four domains (VH, CH1, CH2, and CH3) each of which has one intrachain disulfide bridge.

Depending on the amino acid sequence of the constant (C) domain of the heavy chains, immunoglobulins can be assigned to different classes. There are five major classes of immunoglobulins: IgA, IgD, IgE, IgG, and IgM. The heavy-chain constant domains that correspond to the different classes of immunoglobulins are called α, δ, ε, γ, and μ domains respectively. Sequence studies have shown that the μ chain of IgM contains five domains VH, CHμ1, CHμ2, CHμ3, and CHμ4. The heavy chain of IgE (e) also contains five domains while the heavy chain of IgA (α) has four domains. The immunoglobulin class can be further divided into subclasses (isotypes), e.g., $IgG_1$, $IgG_2$, $IgG_3$, $IgG_4$, $IgA_1$, and $IgA_2$.

The subunit structures and three-dimensional configurations of different classes of immunoglobulins are well known. Of these IgA and IgM are polymeric and each subunit contains two light and two heavy chains. The heavy chain of IgG (γ) contains a length of polypeptide chain lying between the CHγ1 and CHγ2 domains known as the hinge region. The α chain of IgA has a hinge region containing an O-linked glycosylation site and the μ and ε chains do not have a sequence analogous to the hinge region of the γ and α chains, however, they contain a fourth constant domain lacking in the others. The domain composition of immunoglobulin chains can be summarized as follows:

**Light Chain λ = Vλ Cλ**

**κ = Vκ Cκ**

Heavy Chain IgG (γ) = VH CHγ1, hinge CHγ2 CHγ3
IgM (μ) = VH CHμ1 CHμ2 CHμ3 CHμ4
IgA (α) = VH CHα1 hinge CHα2 CHα3
IgE (e) = VH CHε1 CHε2 CHe3 CHε4
IgD (δ) = VH CHδ1 hinge CHδ2 CHδ3

A CH2 domain therefore is an immunoglobulin heavy chain constant region domain. According to the present invention, the CH2 domain is preferably the CH2 domain of one of the five immunoglobulins subtypes indicated above. Preferred are mammalian immunoglobulin CH2 domains such as a primate or murine immunoglobulin with the primate and especially human immunoglobulin CH2 domains being preferred. The amino acid sequence of immunoglobulin CH2 domains are known or are generally available to the skilled artisan (Kabat et al., Sequences of proteins of immunological interest Fifth Ed., U.S. Department of Health and Human Services, NIH Publication No. 91-3242). A preferred immunoglobulin CH2 domain within the context of the present invention is a human IgG and preferably from IgG1, IgG2, IgG3, IgG4 and more preferably a human IgG1. Using the numbering system of Edelman, G.M., et al., (1969) Proc. Natl. Acad. Sci. USA 63:78-85 the immunoglobulin CH2 domain preferably begins at amino position equivalent to glutamine 233 of human IgG1 and extends through amino acid equivalent to lysine 340 (Ellison et al., (1982) EMBO J. 1:403-407).

With respect to human antibody molecules reference is made to the IgG class in which an N-linked oligosaccharide is attached to the amide side chain of Asn 297 of the β-4 bend of the inner face of the CH2 domain of the Fc region (Beale and Feinstein (1976) Q. Rev. Biophys. 9:253-259; Jefferis et al. (1995) Immunol. Letts. 44:111-117). It is characteristic of the glycoprotein of the present invention that it contain or be modified to contain at least a CH2 domain. The CH2 domain is a CH2 domain of an immunoglobulin having a single N-linked oligosaccharide of a human IgG CH2 domain. The CH2 domain is preferably the CHγ2 domain of human $IgG_1$.

The oligosaccharides of the present invention occur on the CH2 domain expressed as N-linked oligosaccharides. "N-linked glycosylation" refers to the attachment of the carbohydrate moiety via GlcNAc to an asparagine residue in a polypeptide chain. The N-linked carbohydrates all contain a common Man 1-6(Man 1-3)Manβ1-4GlcNAcβ1-4GlcNAcβ-R core structure. Therefore, in the core structure described, R represents an asparagine residue of the produced glycoprotein. The sequence of the protein produced will contain an asparagine-X-serine, asparagine-X-threonine, and asparagine-X-cysteine, wherein X is any amino acid except proline. The skilled artisan will recognize that, for example, each of murine IgG3, IgG1, IgG2B, IgG2A and human IgD, IgG3, IgG1, IgA1, IgG2 and IgG4 CH2 domains have a single site for N-linked glycosylation at amino acid residue 297 (Kabat et al., Sequences of proteins of immunological interest Fifth Ed., U.S. Department of Health and Human Services, NIH Publication No. 91-3242).

Of the N-linked carbohydrates the most important are the "complex" N-linked carbohydrates of the variety naturally occurring in immunoglobulin CH2 domains. According to the present invention such complex carbohydrates will be one of the "bi-antennary" structures described herein. The core bi-antennary structure (GlcNAc2Man3GlcNAc) is typical of biantennary oligosaccharides and can be represented schematically as:

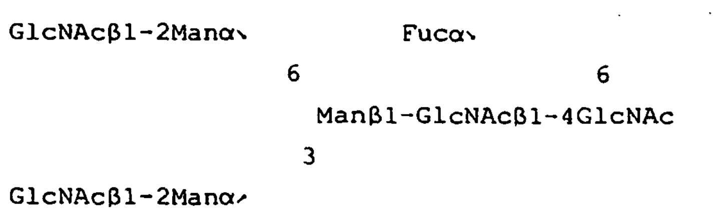

Since each biantennary structure may have a bisecting N-acetylglucoseamine, core fucose and either galactose or sialic acid outer saccharides, there are a total of 36 structurally unique oligosaccharides which may occupy the N-linked Asn 297 site (Jefferis and Lund supra). It will also be recognized that within a particular CH2 domain, glycosylation at Asn 297 may be asymmetric owing to different oligosaccharide chains attached at either Asn 297 residue within the two chain Fc domain. For example, while the heavy chain synthesized within a single antibody-secreting cell may be homogeneous in its amino acid sequence, it is generally differentaly glycosylated resulting in a large number of structurally unique Ig glycoforms.

The major types of complex oligosaccharide structures found in the CH2 domain of the IgG are represented below.

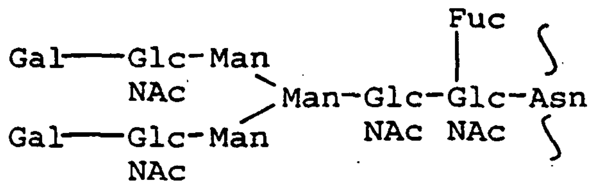
G2

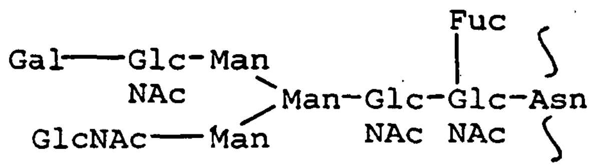
G1

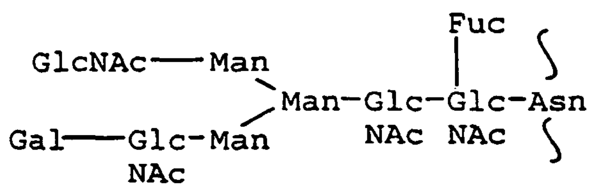
G1

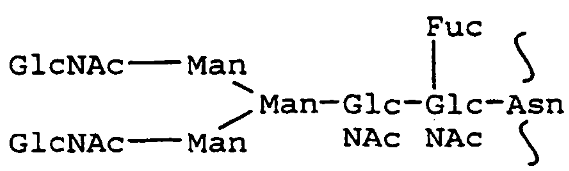
G0

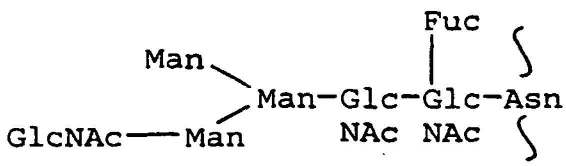

G-1

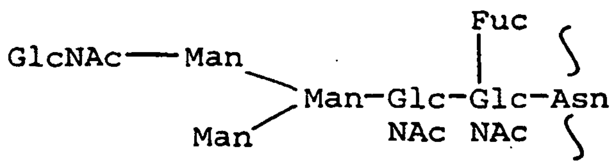

G-1

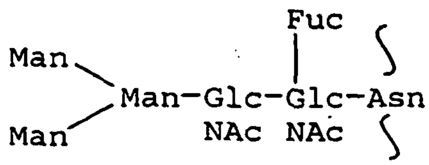

G-2

According to the present invention G0 refers to a biantennary structure wherein no terminal sialic acids (NeuAcs) or Gals are present, G I refers to a biantennary structure having one Gal and no NeuAcs and G2 refers to a biantennary structure with two terminal Gals and no NeuAcs. Accordingly, G-1 refers to the core unit minus one GlcNAc and G-2 refers to the core structure minus two GlcNAc's.

The term "glycoform" as used within the context of the present invention is meant to denote a glycoprotein containing a particular carbohydrate structure or structures. Therefore, the G-2 glycoform of a CH2 domain refers to a CH2 domain having a G-2 glycan as defined herein.

The phrases "substantially homogeneous", "substantially uniform" and "substantial homogeneity" and the like are used to indicate that the product is substantially devoid of by-products originating from undesired glycoforms (e.g. G0 and G1). Expressed in terms of purity, substantial homogeneity means that the amount of by-products does not exceed 10%, and preferably is below 5%, more preferably below 1%, most preferably below 0.5%, wherein the percentages are by weight.

The phrases "substantially free of" and the like are used to indicate that the product is substantially devoid of by-products originating from undesired glycoforms (*e.g.* G0 and G1). Expressed in terms of purity, substantially free means that the amount of by-products does not exceed 10%, and preferably is below 5%, more preferably below 1%, most preferably below 0.5%, wherein the percentages are by weight.

The "CD20" antigen is expressed during early pre-B cell development and may regulate a step in cellular activation required for cell cycle initiation and differentiation. The CD20 antigen is expressed at high levels on neoplastic B cells however it is present on normal B cells as well. Anti-CD20 antibodies which recognize the CD20 surface antigen have been used clinically to lead to the targeting and destruction of neoplastic B cells (Maloney et al., (1994) Blood 84: 2457-2466; Press et al., (1993) NEJM 329:12219-12223; Kaminski et al., (1993) NEJM 329; McLaughlin et al., (1996) Proc. Am. Soc. Clin. Oncol. 15:417). Chimeric and humanized anti-CD20 antibodies mediate complement dependent lysis of target B cells (Maloney et al. supra). The monoclonal antibody C2B8 recognizes the human B cell restricted differentiation antigen Bp35 (Liu et al., (1987) J. Immunol. 139:3521; Maloney et al., (1994) Blood 84:2457). "C2B8" is defined as the anti-CD20 monoclonal antibody described in International Publication No. WO94/11026.

The terms antibody and immunoglobulins are used interchangeably and used to denote glycoproteins having the structural characteristics noted above for immunoglobulins.

The term "antibody" is used in the broadest sense and specifically covers single monoclonal antibodies (including agonist and antagonist antibodies) and antibody compositions with polyepitopic specificity. The term "antibody" specifically covers monoclonal antibodies (including full length monoclonal antibodies), polyclonal antibodies, multispecific antibodies (*e.g.*, bispecific antibodies), and antibody fragments so long as they contain or are modified to contain at least the portion of the CH2 domain of the heavy chain immunoglobulin constant region comprising the singled N-linked glycosylation site. Exemplary antibodies within the scope of the present invention include but are not limited to anti-IL-8, St John et al., (1993) Chest 103:932 and International Publication No. WO 95/23865; anti-CD11a, Filcher et al., Blood, 77:249-256, Steppe et al., (1991) Transplant Intl. 4:3-7, and Hourmant et al., (1994) Transplantation 58:377-380; anti-IgE, Presta et al., (1993) J. Immunol. 151:2623-2632, and International Publication No. WO 95/19181; anti-HER2, Carter et al., (1992) Proc. Natl. Acad. Sci. USA 89:4285-4289, and International Publication No. WO 92/20798; anti-VEGF, Jin Kim et al., (1992) Growth Factors, 7:53-64, and International Publication No. WO 96/30046; and anti-CD20, Maloney et al., (1994) Blood, 84:2457-2466, and Liu et al., (1987) J. Immunol., 130:3521-3526.

The term "preparation" as used herein is used to define a composition which has been identified and separated and/or recovered as component of its environment. Contaminant components of its environment are materials which would interfere with diagnostic or therapeutic uses for the glycoprotein such as unwanted or unintended glycoforms (G0 and G1), and may include enzymes, hormones, and other proteinaceous or nonproteinaceous solutes. The preparation of the invention is substantially free of these contaminants. In preferred embodiments, the glycoprotein preparation will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain.

The term "monoclonal antibody" (mAb) as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each mAb is directed against a single determinant on the antigen. In addition to their specificity, the monoclonal antibodies are advantageous in that they can be synthesized by hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., (1975) Nature, 256:495, or may be made by recombinant DNA methods (see, *e.g.*, U.S. Patent No. 4,816,567 to Cabilly et al.).

The monoclonal antibodies herein include hybrid and recombinant antibodies produced by splicing a variable (including hypervariable) domain of an antibody with a constant domain (e.g. "humanized" antibodies), or a light chain with a heavy chain, or a chain from one species with a chain from another species, or fusions with heterologous proteins, regardless of species of origin or immunoglobulin class or subclass designation, (See, e.g., U.S. Pat. No. 4,816,567 to Cabilly et al.; Mage and Lamoyi, in Monoclonal_Antibody Production Techniques and Applications, pp. 79-97 (Marcel Dekker, Inc., New York, 1987).)

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they contain or are modified to contain at least one CH2 domain (Cabilly *et al.*, supra; Morrison et al., (1984) Proc. Natl. Acad. Sci. U.S.A. 81:6851.

"Humanized" forms of non-human (e.g., murine) antibodies are specific chimeric immunoglobulins, immunoglobulin chains or fragments thereof (such as Fv, Fab, Fab', $F(ab')_2$, or other antigen-binding subsequences of antibodies) which contain minimal sequence derived from non-human immunoglobulin. For the most part, humanized antibodies are human immunoglobulins (recipient antibody) in which residues from a complementary-determining region (CDR) of the recipient are replaced by residues from a CDR of a non-human species (donor antibody) such as mouse, rat, or rabbit having the desired specificity, affinity, and capacity. In some instances, Fv framework residues of the human immunoglobulin are replaced by corresponding non-human residues. Furthermore, humanized antibodies can comprise residues which are found neither in the recipient antibody nor in the imported CDR or framework sequences. These modifications are made to further refine and maximize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin. For further details see Jones et al., Nature 321:522 (1986); Reichmann et al., Nature 332:323 (1988); and Presta, Curr. Op. Struct. Biol. 2:593 (1992).

As used herein, the term "immunoadhesin" designates antibody-like molecules which combine the "binding domain" of a heterologous protein (an "adhesin", *e.g.* a receptor, ligand or enzyme) with the effector functions of immunoglobulin constant domains. Structurally, the immunoadhesins comprise a fusion of the adhesin amino acid sequence with the desired binding specificity which is other than the antigen recognition and binding site (antigen combining site) of an antibody (i.e. is "heterologous") and an immunoglobulin constant domain sequence. The immunoglobulin constant

domain sequence in the immunoadhesin may be obtained from any immunoglobulin, such as $IgG_1$, $IgG_2$, $IgG_3$, or $IgG_4$ subtypes, IgA, IgE, IgD or IgM. Immunoadhesins are described in, for example, U.S. Patent No. 5,116,964.

As used herein the phrase "multispecific immunoadhesin" designates immunoadhesins (as hereinabove defined) having at least two binding specificities (*i.e.* combining two or more adhesin binding domains). Multispecific immunoadhesins can be assembled as heterodimers, heterotrimers or heterotetramers, essentially as disclosed in WO 89/02922 (published 6 April 1989), in EP 314,317 (published 3 May 1989), and in U.S. Patent No. 5,116,964 issued 2 May 1992. Preferred multispecific immunoadhesins are bispecific. Examples of bispecific immunoadhesins include CD4-IgG/TNFreceptor-IgG and CD4-IgG/L-selectin-IgG. The last mentioned molecule combines the lymph node binding function of the lymphocyte homing receptor (LHR, L-selectin), and the HIV binding function of CD4, and finds potential application in the prevention or treatment of HIV infection, related conditions, or as a diagnostic.

An "antibody-immunoadhesin chimera (Ab/Ia chimera)" comprises a molecule which combines at least one binding domain of an antibody (as herein defined) with at least one immunoadhesin (as defined in this application). Exemplary Ab/Ia chimeras are the bispecific CD4-IgG chimeras described by Berg et al., supra and Chamow et al., supra. Immunoadhesons include CD4 (Capon et al., (1989) Nature 337:525-531; Traunecker et al., (1989) Nature 339: 68-70; and Bym et al., (1990) Nature 344:667-670); L-selectin or homing receptor (Watson et al., (1990) J. Cell. Biol. 110:2221-2229; and Watson et al., (1991) Nature 349:164-167); CD44 (Aruffo et al., (1990) Cell 61:1303-1313; CD28 and B7 (Linsley et al., (1991) J. Exp. Med. 173:721-730); CTLA-4 (Lisley et al., J. Exp. Med. 174:561-569); CD22 (Stamenkovic et al., Cell 66:1133-1144); TNF receptor (Ashkenazi et al., (1991) Proc. Natl. Acad. Sci. USA 88: 10535-10539; Lesslauer et al., (1991) Eur. J. Immunol. 27:2883-2886; and Peppel et al., (1991) J. Exp. Med. 174: 1483-1489); NP receptors (Bennett et al., (1991) J. Biol. Chem. 266:23060-23067; interferon $\gamma$ receptor (Kurschner et al., (1992) J. Biol. Chem. 267:9354-9360; 4-IBB (Chalupny et al., (1992) PNAS USA 89:10360-10364) and IgE receptor $\alpha$ (Ridgway and Gorman, (1991) J. Cell. Biol. 115, Abstract No. 1448).

Examples of homomultimeric immunoadhesins which have been described for therapeutic use include the CD4-IgG immunoadhesin for blocking the binding of HIV to cell-surface CD4. Data obtained from Phase I clinical trials in which CD4-IgG was administered to pregnant women just before delivery suggests that this immunoadhesin may be useful in the prevention of maternal-fetal transfer of HIV. Ashkenazi (1991) et al., Intem. Rev. Immunol. 10:219-227. An immunoadhesin which binds tumor necrosis factor (TNF) has also been developed. TNF is a proinflammatory cytokine which has been shown to be a major mediator of septic shock. Based on a mouse model of septic shock, a TNF receptor immunoadhesin has shown promise as a candidate for clinical use in treating septic shock (Ashkenazi, A. et al. (1991) PNAS USA 88:10535-10539).

If the two arms of the immunoadhesin structure have different specificities, the immunoadhesin is called a "bispecific immunoadhesin" by analogy to bispecific antibodies. Dietsch et al., (1993) J. Immunol. Methods 162:123 describe such a bispecific immunoadhesin combining the extracellular domains of the adhesion molecules, E-selectin and P-selectin, each of which selectins is expressed in a different cell type in nature. Binding studies indicated that the bispecific immunoglobulin fusion protein so formed had an enhanced ability to bind to a myeloid cell line compared to the monospecific immunoadhesins from which it was derived.

The invention also pertains to immunoconjugates comprising the antibody described herein conjugated to a cytotoxic agent such as a chemotherapeutic agent, toxin (*e.g.*, an enzymatically active toxin of bacterial, fungal, plant or animal origin, or fragments thereof), or a radioactive isotope (*i.e.*, a radioconjugate).

In another embodiment, the antibody may be conjugated to a "receptor" (such as streptavidin) for utilization in tumor pretargeting wherein the antibody-receptor conjugate is administered to the patient, followed by removal of unbound conjugate from the circulation using a clearing agent and then administration of a "ligand" (*e.g.*, avidin) which is conjugated to a cytotoxic agent (*e.g.*, a radionuclide).

"Treatment" refers to both therapeutic treatment and prophylactic or preventative measures. Those in need of treatment include those already with the disorder as well as those prone to have the disorder or those in which the disorder is to be prevented.

The terms "treating," "treatment," and "therapy" refer to curative therapy, prophylactic therapy, and preventative therapy.

The term "mammal" refers to any animal classified as a mammal, including humans, cows, horses, dogs and cats. In a preferred embodiment of the invention, the mammal is a human.

As used herein, protein, peptide and polypeptide are used interchangeably to denote an amino acid polymer or a set of two or more interacting or bound amino acid polymers.

The term "disease state" refers to a physiological state of a cell or of a whole mammal in which an interruption, cessation, or disorder of cellular or body functions systems, or organs has occurred.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia. More particular examples of such cancers include squamous cell cancer, small-cell lung cancer, non-small cell lung cancer, gastric cancer, pancreatic cancer, glial cell tumors such as glioblastoma and

neurofibromatosis, cervical cancer, ovarian cancer, liver cancer, bladder cancer, hepatoma, breast cancer, colon cancer, colorectal cancer, endometrial carcinoma, salivary gland carcinoma, kidney cancer, renal cancer, prostate cancer, vulval cancer, thyroid cancer, hepatic carcinoma and various types of head and neck cancer.

The term "inflammatory disorder" refers to a fundamental pathologic process consisting of a dynamic complex of cytologic and histologic reactions that occur in the affected blood vessels and adjacent tissues in response to an injury or abnormal stimulation caused by a physical, chemical, or biologic agent, including: 1) the local reactions and resulting morphologic changes, 2) the destruction or removal of the injurious material, 3) the responses that lead to repair and healing. Inflammatory disorders treatable by the invention are those wherein the inflammation is associated with cytokine-induced disorders, such as those associated with interleukin and leukemia inhibitory factor cytokines. Such disorders include abnormalities in thrombopoiesis, macrophage growth and differentiation, proliferation of hematopoietic progenitors, and the like.

The term "neurological disorder" refers to or describes the physiological condition in mammals that is typically characterized by nerve cell growth, differentiation, or cell signaling. Examples of neurological disorders include, but are not limited to, neurofibromatosis and peripheral neuropathy.

The term "cardiac disorder" refers to or describes the physiological condition in mammals that is typically characterized by cardiac cell growth and differentiation. An example of a cardiac disorder includes, but is not limited to, cardiac hypertrophy and heart failure, including congestive heart failure, myocardial infarction, and tachyrhythmia.. "Heart failure" refers to an abnormality of cardiac function where the heart does not pump blood at the rate needed for the requirements of metabolizing tissues.

## Modes for Carrying out the Invention

The compositions and preparations of the present invention are preferably obtained by in vitro modification of recombinantly produced glycoproteins. The skilled artisan will recognize that both the structure of the attached-oligosaccharide and the efficiency of glycosylation of a glycoprotein produced in recombinant cell culture will vary depending upon the method of glycoprotein production employed. Oligosaccharide structures attached at particular glycosylation sites will generally vary even for monoclonal antibodies. Therefore, it is typical to find multiple glycoforms within a given production or batch for monoclonal as well as polyclonal antibodies.

According to the invention, a compositions is prepared comprising a glycoprotein having an immunoglobulin CH2 domain wherein the CH2 domain has at least one N-linked oligosaccharide wherein substantially all of the oligosaccharide of the CH2 domain is a G-2 oligosaccharide as defined herein. The invention further provides for the preparation of a compositions comprising a glycoprotein having at least one immunoglobulin CH2 domain wherein the N-linked oligosaccharides of the CH2 domain are substantially of the G2 and G-2 variety. The composition is substantially free of glycoproteins comprising an immunoglobulin CH2 domain wherein the N-linked oligosaccharide is a G 1, G0 or G-1 oligosaccharide.

The present invention provides that a substantially homogenous glycoform can be obtained and that, according to certain embodiments, the glycoform exhibits a favorable bioactivity compared with the heterogenous glycoform. According to the present invention a substantially homogenous glycoform of a glycoprotein comprising a CH2 domain, such as an antibody, is obtained. Compositions comprising the glycoprotein glycoform are prepared which are substantially free of undesired glycoforms. Therefore, according to one aspect of the present invention, a composition comprising a glycoprotein having a CH2 domain such as, for example, an IgG1 type antibody containing a single site for N-linked glycosylation in each CH2 domain and having a particular N-linked oligosaccharide is prepared. In one embodiment the antibody glycoform is a G2 glycoform. In a further embodiment, the antibody is a G-2 glycoform. A further aspect of the present invention provides for a composition as described, being substantially free of G1, G0 and G-1 antibody glycoforms. This composition combines the G2 and G-2 antibody glycoforms of the present invention.

The glycoproteins, for example antibodies of the present invention can be produced by well known techniques including but not limited to gene expression systems to allow the production of intact glycoproteins comprising a CH2 domain in any of a variety of host systems. Both prokaryotic and eukaryotic expression systems, for example can be used in the production of the glycoproteins of the present invention however, eukaryotic expression systems are preferred since antibodies produced in prokaryotic cell systems lack carbohydrate.

### Isolating Antibodies

Techniques for isolating antibodies and preparing immunoadhesins follow. However, it will be appreciated that the glycoprotein can be isolated using techniques which are known in the art.

(i) Antibody preparation

Several techniques for the production of antibodies have been described which include the traditional hybridoma method for making monoclonal antibodies, recombinant techniques for making antibodies (including chimeric antibodies, *e.g.* humanized antibodies), antibody production in transgenic animals and the recently described phage display technology for preparing "fully human" antibodies. These techniques shall be described briefly below.

Polyclonal antibodies to the antigen of interest generally can be raised in animals by multiple subcutaneous (sc) or intraperitoneal (ip) injections of the antigen and an adjuvant. It may be useful to conjugate the antigen (or a fragment containing the target amino acid sequence) to a protein that is immunogenic in the species to be immunized, e.g., keyhole limpet hemocyanin, serum albumin, bovine thyroglobulin, or soybean trypsin inhibitor using a bifunctional or derivatizing agent, for example maleimidobenzoyl sulfosuccinimide ester (conjugation through cysteine residues), N-hydroxysuccinimide (through lysine residues), glutaraldehyde, succinic anhydride, $SOCl_2$, or $R^1N=C=NR$, where R and $R^1$ are different alkyl groups. Animals are immunized against the immunogenic conjugates or derivatives by combining 1 mg of 1 $\mu$g of conjugate (for rabbits or mice, respectively) with 3 volumes of Freud's complete adjuvant and injecting the solution intradermally at multiple sites. One month later the animals are boosted with 1/5 to 1/10 the original amount of conjugate in Freud's complete adjuvant by subcutaneous injection at multiple sites. 7 to 14 days later the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are used to enhance the immune response.

Monoclonal antibodies are obtained from a population of substantially homogeneous antibodies using the hybridoma method first described by Kohler & Milstein, (1975) Nature 256:495 or may be made by recombinant DNA methods (Cabilly et al., U.S. Patent No. 4,816,567). In the hybridoma method, a mouse or other appropriate host animal, such as hamster, is immunized as hereinabove described to elicit lymphocytes that produce, or are capable of producing, antibodies that will specifically bind to the protein used for immunization. Alternatively, lymphocytes may be immunized *in vitro*. Lymphocytes then are fused with myeloma cells using a suitable fusing agent, such as polyethylene glycol, to form a hybridoma cell (Goding, Monoclonal Antibodies: Principles and Practice, pp.59-103 [Academic Press, 1986]). The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells. Preferred myeloma cells are those that fuse efficiently, support stable high level expression of antibody by the selected antibody-producing cells, and are sensitive to a medium such as HAT medium. Among these, preferred myeloma cell lines are murine myeloma lines, such as those derived from MOPC-21 and MPC-11 mouse tumors available from the Salk Institute Cell Distribution Center, San Diego, California USA, and SP-2 cells available from the American Type Culture Collection, Rockville, Maryland USA. Human myeloma and mouse-human heteromyeloma cell lines also have been described for the production of human monoclonal antibodies (Kozbor, (1984) J. Immunol., 133:3001; and Brodeur et al., Monoclonal Antibody Production Techniques and Applications, pp. 51-63, Marcel Dekker, Inc., New York, 1987). See, also, Boerner et al., (1991) J. Immunol., 147(1):86-95 and WO 91/17769, published Nov 28, 1991, for techniques for the production of human monoclonal antibodies. Culture medium in which hybridoma cells are growing is assayed for production of monoclonal antibodies directed against the antigen of interest. Preferably, the binding specificity of monoclonal antibodies produced by hybridoma cells is determined by immunoprecipitation or by an *in vitro* binding assay, such as radioimmunoassay (RIA) or enzyme-linked immunoabsorbent assay (ELISA). The binding affinity of the monoclonal antibody can, for example, be determined by the Scatchard analysis of Munson & Pollard, (1980) Anal. Biochem. 107:220. After hybridoma cells are identified that produce antibodies of the desired specificity, affinity, and/or activity, the clones may be subcloned by limiting dilution procedures and grown by standard methods. Goding, Monoclonal Antibodies: Principles and Practice, pp.59-104 (Academic Press, 1986). Suitable culture media for this purpose include, for example, Dulbecco's Modified Eagle's Medium or RPMI-1640 medium. In addition, the hybridoma cells may be grown in vivo as ascites tumors in an animal. The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

Alternatively, it is now possible to produce transgenic animals (e.g. mice) that are capable, upon immunization, of producing a full repertoire of human antibodies in the absence of endogenous immunoglobulin production. For example, it has been described that the homozygous deletion of the antibody heavy chain joining region ($J_H$) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, *e.g.*, Jakobovits et al. (1993) Proc. Natl. Acad. Sci. USA 90:2551-255 and Jakobovits et

al., (1993) Nature 362:255-258.

In a further embodiment, antibodies or antibody fragments can be isolated from antibody phage libraries generated using the techniques described in McCafferty et al., (1990) Nature, 348:552-554 (1990), using the antigen of interest to select for a suitable antibody or antibody fragment. Clackson et al., (1991) Nature, 352:624-628 (1991) and Marks et al., (1991) J. Mol. Biol., 22:581-597 describe the isolation of murine and human antibodies, respectively, using phage libraries. Subsequent publications describe the production of high affinity (nM range) human antibodies by chain shuffling (Mark et al., (1992) Bio/Technol. 10:779-783), as well as combinatorial infection and in vivo recombination as a strategy for constructing very large phage libraries (Waterhouse et al., (1993) Nuc. Acids Res., 21:2265-2266). Thus, these techniques are viable alternatives to traditional monoclonal antibody hybridoma techniques for isolation of "monoclonal" antibodies (especially human antibodies) which are encompassed by the present invention.

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al., (1986) Nature 321:522-525; Riechmann et al., (1988) Nature 332:323-327; Verhoeyen et al., (1986) Science 239:1534-1536), by substituting rodent CDRs or CDR sequences for the corresponding sequences of a human antibody. Accordingly, such "humanized" antibodies are chimeric antibodies (Cabilly, *supra*), wherein substantially less than an intact human variable domain has been substituted by the corresponding sequence from a non-human species. In practice, humanized antibodies are typically human antibodies in which some CDR residues, and possibly some FR residues, are substituted by residues from analogous sites in rodent antibodies. It is important that antibodies be humanized with retention of high affinity for the antigen and other favorable biological properties. To achieve this goal, according to a preferred method, humanized antibodies are prepared by a process of analysis of the parental sequences and various conceptual humanized products using three dimensional models of the parental and humanized sequences. Three dimensional immunoglobulin models are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, *i.e.*, the analysis of residues that influence the ability of the candidate immunoglobulin to bind its antigen. In this way, FR residues can be selected and combined from the consensus and import sequence so that the desired antibody characteristic, such as increased affinity for the target antigen(s), is achieved. For further details see WO 92/22653, published Dec 23, 1992.

Immunoglobulins (Ig) and certain variants thereof are known and many have been prepared in recombinant cell culture. For the antibodies described above, the use of human IgG immunoglobulin sequences is preferred since this structure contains the CH2 domain of the present invention. For example, see U.S. Patent No. 4,745,055; EP 256,654; Faulkner et al., (1982) Nature 298:286 ; EP 120,694; EP 125,023; Morrison, J. Immun. 123:793 (1979); Köhler et al., (1980) Proc. Natl. Acad. Sci. USA 77:2197; Raso et al., (1981) Cancer Res. 41:2073; Morrison et al., (1984) Ann. Rev. Immunol. 2:239; Morrison, (1985) Science 229:1202; Morrison et al., (1984) Proc. Natl. Acad. Sci. USA 81:6851; EP 255,694; EP 266,663; and WO 88/03559.

Preferred antibodies within the scope of the present invention include anti-IL-8 (St John et al., (1993), Chest, 103:932 and International Publication No. WO 95/23865); anti-CD11a (Filcher et al., Blood, 77:249-256, Steppe et al., (1991), Transplant Intl. 4:3-7, and Hourmant et al., (1994), Transplantation 58:377-380); anti-IgE (Presta et al., (1993), J. Immunol. 151:2623-2632, and International Publication No. WO 95/19181); anti-HER2 (Carter et al., (1992), Proc. Natl. Acad. Sci. USA, 89:4285-4289, and International Publication No. WO 92/20798); anti-VEGF (Jin Kim et al., (1992) Growth Factors, 7:53-64, and International Publication No. WO 96/30046); and anti-CD20 (Maloney et al., (1994) Blood, 84:2457-2466, Liu et al., (1987) J. Immunol., 130:3521-3526).

(ii) Immunoadhesin preparation

Chimeras constructed from an adhesin binding domain sequence linked to an appropriate immunoglobulin constant domain sequence (immunoadhesins) are known in the art. Immunoadhesins reported in the literature include fusions of CD4 (Capon et al., (1989) Nature 337:525-531; Traunecker et al., (1989) Nature 339:68-70; Zettmeissl et al., (1990) DNA Cell Biol. USA 9:347-353; and Byrn et al.,(1990) Nature 344:667-670); L-selectin (homing receptor) (Watson et al., (1990) J. Cell. Biol. 110:2221-2229; and Watson et al., (1991) Nature 349:164-167); CD44 (Aruffo et al., (1990) Cell 61:1303-1313); CD28 and B7 (Linsley et al., (1991) J. Exp. Med. 173:721-730); CTLA-4 (Lisley et al., (1991) J. Exp. Med. 174:561-569); CD22 (Stamenkovic et al., (1991) Cell 66:1133-1144); TNF receptor (Ashkenazi et al.,(1991) Proc. Natl. Acad. Sci. USA 88:10535-10539; Lesslauer et al., (1991) Eur. J. Immunol. 27:2883-2886; and Peppel et al., (1991) J. Exp. Med. 174:1483-1489); and IgE receptor a (Ridgway and Gorman, (1991) J. Cell. Biol. 115: Abstract No. 1448).

Typically, in such fusions the encoded chimeric polypeptide will retain at least functionally active hinge, CH2 and CH3 domains of the constant region of an immunoglobulin heavy chain. Fusions are also made to the C-terminus

of the Fc portion of a constant domain, or immediately N-terminal to the CH1 of the heavy chain or the corresponding region of the light chain. The precise site at which the fusion is made is not critical; particular sites are well known and may be selected in order to optimize the biological activity, secretion, or binding characteristics of the Ia.

In a preferred embodiment, the adhesin sequence is fused to the N-terminus of the Fc domain of immunoglobulin $G_1$ ($IgG_1$). It is possible to fuse the entire heavy chain constant region to the adhesin sequence. However, more preferably, a sequence beginning in the hinge region just upstream of the papain cleavage site which defines IgG Fc chemically (*i.e*. residue 216, taking the first residue of heavy chain constant region to be 114), or analogous sites of other immunoglobulins is used in the fusion. In a particularly preferred embodiment, the adhesin amino acid sequence is fused to (a) the hinge region and CH2 and CH3 or (b) the CH1, hinge, CH2 and CH3 domains, of an $IgG_1$ heavy chain. The precise site at which the fusion is made is not critical, and the optimal site can be determined by routine experimentation.

For bispecific immunoadhesins, the immunoadhesins are assembled as multimers, and particularly as heterodimers or heterotetramers. Generally, these assembled immunoglobulins will have known unit structures. A basic four chain structural unit is the form in which IgG, IgD, and IgE exist. A four chain unit is repeated in the higher molecular weight immunoglobulins; IgM generally exists as a pentamer of four basic units held together by disulfide bonds. IgA globulin, and occasionally IgG globulin, may also exist in multimeric form in serum. In the case of multimer, each of the four units may be the same or different.

Various exemplary assembled immunoadhesins within the scope herein are schematically diagramed below:

(a) $AC_H$-[$AC_H$, $AC_L$-$AC_H$, $AC_L$-$V_HC_H$, or $V_LC_L$-$AC_H$];
(b) $AC_L$-$AC_H$-[$AC_L$-$AC_H$, $AC_L$-$V_HC_H$, $V_LC_L$-$AC_H$, or $V_LC_L$-$V_HC_H$];
(c) $AC_L$-$V_HC_H$-[$AC_H$, or $AC_L$-$V_HC_H$, or $V_LC_L$-$AC_H$];
(d) $V_LC_L$-$AC_H$-($AC_L$-$V_HC_H$, or $V_LC_L$-$AC_H$]; and
(e) $[A\text{-}Y]_n$-$[V_LC_L\text{-}V_HC_H]_2$,

wherein each A represents identical or different adhesin amino acid sequences;

$V_L$ is an immunoglobulin light chain variable domain;
$V_H$ is an immunoglobulin heavy chain variable domain;
$C_L$ is an immunoglobulin light chain constant domain;
$C_H$ is an immunoglobulin heavy chain constant domain;
n is an integer greater than 1;
Y designates the residue of a covalent cross-linking agent.

In the interests of brevity, the foregoing structures only show key features; they do not indicate joining (J) or other domains of the immunoglobulins, nor are disulfide bonds shown. However, where such domains are required for binding activity, they shall be constructed to be present in the ordinary locations which they occupy in the immunoglobulin molecules.

Alternatively, the adhesin sequences can be inserted between immunoglobulin heavy chain and light chain sequences, such that an immunoglobulin comprising a chimeric heavy chain is obtained. In this embodiment, the adhesin sequences are fused to the 3' end of an immunoglobulin heavy chain in each arm of an immunoglobulin, either between the hinge and the CH2 domain, or between the CH2 and CH3 domains. Similar constructs have been reported by Hoogenboom, et al., (1991) Mol. Immunol, 28:1027-1037).

Although the presence of an immunoglobulin light chain is not required in the immunoadhesins of the present invention, an immunoglobulin light chain might be present either covalently associated to an adhesin-immunoglobulin heavy chain fusion polypeptide, or directly fused to the adhesin. In the former case, DNA encoding an immunoglobulin light chain is typically coexpressed with the DNA encoding the adhesin-immunoglobulin heavy chain fusion protein. Upon secretion, the hybrid heavy chain and the light chain will be covalently associated to provide an immunoglobulin-like structure comprising two disulfide-linked immunoglobulin heavy chain-light chain pairs. Methods suitable for the preparation of such structures are, for example, disclosed in U.S. Patent No. 4,816,567, issued 28 March 1989.

Immunoadhesins are most conveniently constructed by fusing the cDNA sequence encoding the adhesin portion in-frame to an Ig cDNA sequence. However, fusion to genomic Ig fragments can also be used (see, e.g. Aruffo et al., (1990) Cell 61:1303-1313; and Stamenkovic et al., (1991) Cell 66:1133-1144). The latter type of fusion requires the presence of Ig regulatory sequences for expression. cDNAs encoding IgG heavy-chain constant regions can be isolated based on published sequences from cDNA libraries derived from spleen or peripheral blood lymphocytes, by hybridization or by polymerase chain reaction (PCR) techniques. The cDNAs encoding the "adhesin" and the Ig parts of the immunoadhesin are inserted in tandem into a plasmid vector that directs efficient expression in the chosen host cells.

In a preferred embodiment, the immunoglobulin sequences used in the construction of the glycoproteins such as the antibodies and immunoadhesins of the present invention are from an IgG immunoglobulin heavy chain CH2

constant domain. For example, the use of human $IgG_1$ immunoglobulin sequences is preferred because this structure contains the preferred CH2 domain of the present invention. A major advantage of using $IgG_1$ is that IgG1 can be purified efficiently on immobilized protein A. In contrast, purification of $IgG_3$ requires protein G, a significantly less versatile medium. However, other structural and functional properties of immunoglobulins should be considered when choosing the Ig CH2 domain for a particular glycoprotein. For example, the $IgG_3$ hinge is longer and more flexible, so it can accommodate larger "adhesin" domains that may not fold or function properly when fused to $IgG_1$. Another consideration may be valency; IgG immunoadhesins are bivalent homodimers, whereas Ig subtypes like IgA and IgM may give rise to dimeric or pentameric structures, respectively, of the basic Ig homodimer unit. For antibodies and immunoadhesins designed for in vivo application, the pharmacokinetic properties and the effector functions specified by the Fc region are important as well. Although $IgG_1$, $IgG_2$ and $IgG_4$ all have in vivo half-lives of 21 days, their relative potencies at activating the complement system are different. $IgG_4$ does not activate complement, and $IgG_2$ is significantly weaker at complement activation than $IgG_1$. Moreover, unlike $IgG_1$, $IgG_2$ does not bind to Fc receptors on mononuclear cells or neutrophils. this may be due to the differences in CH2 domains utilized in these isotypes. While $IgG_3$ is optimal for complement activation, its in vivo half-life is approximately one third of the other IgG isotypes. Another important consideration for immunoadhesins designed to be used as human therapeutics is the number of allotypic variants of the particular isotype. In general, IgG isotypes with fewer serologically-defined allotypes are preferred. For example, $IgG_1$ has only four serologically-defined allotypic sites, two of which (G1m and 2) are located in the Fc region; and one of these sites, G1m1, is non-immunogenic. In contrast, there are 12 serologically-defined allotypes in IgG3, all of which are in the Fc region; only three of these sites (G3m5, 11 and 21) have one allotype which is nonimmunogenic. Thus, the potential immunogenicity of a γ3 immunoadhesin is greater than that of a γ1 immunoadhesin. Preferred among the immunoadhesins are those comprising at least the IgG1 CH2 domain as described herein above.

Preparing the Glycoprotein

DNA encoding the glycoproteins of the invention is readily isolated and sequenced using conventional procedures (*e.g.*, by using oligonucleotide probes that are capable of binding specifically to genes encoding the heavy and light chains of murine antibodies). The hybridoma cells of the invention serve as a preferred source of such DNA Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of the glycoprotein, for example monoclonal antibodies, in the recombinant host cells.

Various techniques for making and isolating antibody and immunoadhesins and the like directly from recombinant cell culture have also been described. In particular, the cells which express the desired glycoprotein should express or be manipulated to express the particular enzymes such that under the appropriate conditions, the appropriate post-translational modification occurs in vivo. The enzymes include those enzymes necessary for the addition and completion ofN- and O- linked carbohydrates such as those described in Hubbard and Ivan supra for N-linked oligosaccharides. The enzymes optionally include oligosaccharyltransferase, alpha-glucosidase I, alpha-glucosidase II, ER alpha (1,2)mannosidase, Golgi alpha-mannosidase I, N-acetylyglucosaminyltransferase I, Golgi alpha-mannosidase II, N-acetylyglucosaminyltransferase II, alpha(1,6)fucosyltransferase, and β(1,4)galactosyltransferase

Typically, the cells are capable of expressing and secreting large quantities of a particular glycoprotein of interest into the culture medium. Examples of suitable mammalian host cells within the context of the present invention may include Chinese hamster ovary cells/-DHFR (CHO, Urlaub and Chasin, Proc. Natl. Acad. Sci. USA, 77:4216 [1980]); dp12.CHO cells (EP 307,247 published 15 March 1989); monkey kidney CV1 line transformed by SV40 (COS-7, ATCC CRL 1651); human embryonic kidney line (293 or 293 cells subcloned for growth in suspension culture, Graham et al., J. Gen Virol., 36:59 [1977]); baby hamster kidney cells (BHK, ATCC CCL 10); mouse sertoli cells (TM4, Mather, Biol. Reprod., 23:243-251 [1980]); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci., 383: 44-68 [1982]); MRC 5 cells; FS4 cells; and a human hepatoma line (Hep G2).

Preferred host cells include Chinese hamster ovary cells/-DHFR (CHO, Urlaub and Chasin, Proc. Natl. Acad. Sci. USA, 77:4216 [1980]); dp12.CHO cells (EP 307,247 published 15 March 1989).

For the culture of the mammalian cells expressing the desired protein and capable of adding the desired carbohydrates at specific positions, numerous culture conditions can be used paying particular attention to the host cell being cultured. Suitable culture conditions for mammalian cells are well known in the art (J. Immunol. Methods (1983) 56:221-234) or can be easily determined by the skilled artisan (see, for example, Animal Cell Culture: A Practical Approach 2nd Ed., Rickwood, D. and Hames, B.D., eds. Oxford University Press, New York (1992)), and vary according to the particular host cell selected.

The glycoprotein of interest preferably is recovered from the culture medium as a secreted polypeptide, although

it also may be recovered from host cell lysates.

As a first step, the culture medium or lysate is centrifuged to remove particulate cell debris. The polypeptide thereafter is purified from contaminant soluble proteins and polypeptides, with the following procedures being exemplary of suitable purification procedures: by fractionation on immunoaffinity or ionexchange columns; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; and protein A Sepharose columns to remove contaminants such as IgG. A protease inhibitor such as phenyl methyl sulfonyl fluoride (PMSF) also may be useful to inhibit proteolytic degradation during purification. One skilled in the art will appreciate that purification methods suitable for the polypeptide of interest may require modification to account for changes in the character of the polypeptide upon expression in recombinant cell culture.

Especially preferred within the context of the present invention are purification techniques and processes which select for the carbohydrates of the invention.

Preparation of G2 Glycoforms

The attachment of a galactose residue to an existing glycan involves the transfer of a galactose moiety from an activated galactose containing compound to the glycosyl moiety of the CH2 domain. The transfer of galactose is catalyzed by a galactosyltransferase enzyme.

While the skilled artisan will recognize that any of several art standard procedures can be employed for the addition of a sugar to a preexisting oligosaccharide chain, the invention preferably utilizes those procedures that result in complete galactosylation of the sample as described herein. By complete galactosylation of the sample is meant that each antennary structure of the native biantennary oligosaccharide terminates in a galactose residue. More particularly the reaction is complete if substantially all N-linked oligosaccharides are of the G2 variety.

According to the present invention a method for producing the compositions of the invention comprising the steps of reacting in an aqueous buffered solution at a temperature of about 25-40 C;

a) a metal salt at a concentration of about 5 mM to about 25 mM;
b) an activated galactose at a concentration of about 5 mM to about 50 mM;
c) a galactosyltransferase at a concentration of about I mUnit/ml to about 100 mUnit/ml; and
d) a substrate glycoprotein; and

recovering the glycoprotein.

As used herein the term galactose (gal) and galactose residue and the like refer to D and L (+/-) galactose. Preferably the gal is D-(+)-galactose which has been reported as a naturally occurring gal in various animal species.

The activated galactose containing compound is generally a uridine diphosphate (UDP)-galactose. Uridine diphosphate-galactose and other donor sugars, which are capable of transferring galactose to N-linked oligosaccharides.

Metal salts include for example, MnCl2, BaCl2, CaCl2, and others.

The galactosyl transferase used in accordance with the present invention is preferably a β1-4 transferase and catalyzes the transfer of a galactose moiety from the activated substrate to the glycosyl compound. The galactosyltransferase enzymes are substrate specific and are named according to their substrate specificity. The galactosyltransferase designated beta 1-4 refers to a galactosyl transferase that catalyzes the transfer of galactose to the hydroxyl group of a glycosyl acceptor compound. Exemplary galactosyltransferases useful within the context of the present invention are from human, bovine, mouse, hamster, or, monkey origin.

Galactosyltransferases are commercially available (Sigma Chemical Co., St. Louis, Mo.; Boehringer Mannheim, Indianapolis, Ind. and Genzyme, Cambridge MA). Alternatively galactosyl transferases are isolated and purified from animal tissue such as bovine (Boeggeman et al., (1993) Prot. Eng. 6(7):779-785;human Schweinteck (1994) Gene 145 (2):299-303; Kleene et al., (1994) Biochem. Biophys. Res. Commun. 201(1):160-167; Chatterjee et al., (1995) Int. J. Biochem Cell. Biol. 27(3):329-336; Herrmann et al., (1995) Protein. Expr. Purif. 6(1):72-78).

The concentration and amount of the various reactants described above depend upon a number of factors including reaction conditions such as temperature and pH and the amount of glycoprotein to be galactosylated. While the present method is thought to be generally applicable to all glycoproteins preferred glycoproteins for use in the present method are glycoproteins comprising at least the CH2 domain of immunoglobulins as described above.

The galactosyltransferase is used in a catalytic amount. By catalytic amount is meant an amount of galactosyltransferase at least sufficient to catalyze in a non-rate-limiting manner the conversion of the enzyme's substrate to product. The catalytic amount of a particular enzyme varies according to the amount of a particular enzyme substrate as well as reaction conditions such as temperature, time and pH value. Enzyme amounts are generally expressed in activity units. One unit catalyzes the formation of 1 μmol of product at a given temperature (typically 37° C) and pH value (typically 7.5) per minute. Thus 10 units of an enzyme is the catalytic amount of that enzyme such that 10 μmol of

substrate are converted to 10 μmols of product in one minute at a temperature of 37° C and a pH of 6.5 to 7.5.

The reaction comprises mixing at least the above ingredients in a suitable aqueous environment to form a reaction mixture and maintaining the reaction mixture under the conditions of temperature, pH, osmolality, ionic composition and ambient temperature for a period of time sufficient to complete the reaction.

The selection of particular conditions depends primarily upon the amount of glycoprotein present. The temperature can range from about 20 C to about 40 C. Preferably the temperature ranges from about 25 to about 40 C. The pH value can range from about 6.0 to about 11.0 preferably the pH value is from about 6.5 to about 8.5 and more preferably about 7.5. The pH is maintained by the addition of a suitable buffer to the reaction. The buffer is devoid of phosphate, EDTA, EGTA and other chelators that bind Mg++ or Mn++. The selection of buffer is based upon the ability of the buffer to maintain the pH at about the desired pH level. Where the pH value is 7.5 the preferred buffers are sodium cacodylate and MES.

In an exemplary method, the glycoprotein samples (e.g. C2B8, anti-HER2, anti-VEGF, anti-IgE and TNFR-IgG) at 10 mg in 0.5 ml, are buffer exchanged into 50 mM sodium cacodylate buffer, pH 7.1 (final vol. 1.0 ml). 50 μl each of 100 mM UDP-Gal and 100 mM $MnCl_2$ are added to the glycoprotein solution. The β1,4-galactosyltransferase (β1,4GT; lyophilized powder) is reconstituted in 50 mM sodium cacodylate buffer, pH 7.1, at a concentration of 1 mU/ml. 50 μl of this solution is added to the reaction mixture and incubated at 37°C for 48 hr. The reaction is stopped by cooling the reaction vial on ice (4°C) for 10 min and the galactosylated antibody is purified on a protein A column.

Preparation of G-2 Glycoforms

The removal of a galactose and an N-acetylglucosamine residue from an existing glycan can be accomplished by methods known to those skilled in the art and involves the use of appropriate enzymes for the removal of the particular residue. Removal of galactose residues is accomplished using galactosidases that recognize the terminal galactose as substrate. Appropriate galactosidases are known to those skilled in the art and include β-D-galctosidases form *Diplococcus pneumoniae*, jack bean, bovine testis, anc chicken liver.

Likewise, removal of terminal N-acetylglucosamine structures can be accomplished using appropriate N-acetylglucosaminidases available to the skilled artisan. Appropriate enzymes for the removal of N-acetylglucasamine terminal residues include N-acetyl β-D-glucosamindases from *Diplococcus pneumoniae,* jack bean, and bovine testis and chicken liver.

While the skilled artisan will recognize that any of several art standard procedures can be employed for the removal of a sugar from a preexisting oligosaccharide chain, the invention preferably utilizes a procedures that results in complete degalactosylation of the sample as well as complete removal of N-acerylglucosamine. By complete degalactosylation of the sample is meant that each antennary structure of the native biantennary oligosaccharide terminates in an N-acetylglucosamine residue following the removal procedure. More particularly the reaction is complete if substantially all N-linked oligosaccharides are of the G0 variety following treatment. Likewise, by complete removal of N-acerylglucoseamine is meant that each antennary structure of the native biantennery oligosaccharide terminates with a mannose residue following the removal procedure. More particularly the reaction is complete if substantially all N-linked oligosaccharides are of the G-2 variety following treatment.

An exemplary procedure employs two appropriate enzymes in a single step procedure. For example, a glycoprotein comprising a CH2 domain such as an antibody (5-10 mg) is buffer-exchanged into 100 mM citrate-phosphate buffer, pH 5.0, using NAP-5 columns (Pharmacia). β-Galactosidase (40 mU/mg protein, *Diplococcus pneumoniae*, Boehringer Mannheim) and N-acetyl-β-D-glucosaminidase (40 mU/mg protein, *Diplococcus pneumoniae* Boehringer Mannheim or jack bean enzyme from Sigma) are added to 5-10 mg aliquots of protein and incubated at 37°C for 12-24 hrs. The IgG samples are purified on a Protein A column. The purified protein is subjected to electrospray ionization mass spectrometry, capillary electrophoresis for carbohydrate analysis to confirm complete removal of the galactose and N-acetylglucosamine residues.

Analysis of the Glycoprotein

The complex carbohydrate portion of the glycoprotein produced by the processes of the present invention may be readily analyzed to determine that the reaction described above is complete. The oligosaccharide are analyzed by conventional techniques of carbohydrate analysis such as those described in the accompanying Figures and Examples. Thus, for example, techniques such as lectin blotting, well-known in the art, reveal proportions of terminal mannose or.other sugars such as galactose.

The carbohydrate structures of the present invention occur on the protein expressed as G2 or G-2 N-linked oligosaccharides. Several methods are known in the art for glycosylation analysis and are useful in the context of the present invention. Such methods provide information regarding the identity and the composition of the oligosaccharide attached to the peptide. Methods for carbohydrate analysis useful in the present invention include but are not limited to

lectin chromatography; HPAEC-PAD, which uses high pH anion exchange chromatography to separate oligosaccharides based on charge; NMR; Mass spectrometry; HPLC; GPC; monosaccharide compositional analysis; sequential enzymatic digestion.

Additionally, methods for releasing oligosaccharides are known. These methods include 1)enzymatic, which is commonly performed using peptide-N-glycosidase F/endo-β-galactosidase; 2) elimination using harsh alkaline environment to release mainly 0-linked structures; and 3) chemical methods using anhydrous hydrazine to release both N- and O-linked oligosaccharides

Analysis can be performed using the following steps:

1. Dialysis of the sample against deionized water, to remove all buffer salts, followed by lyophilization.
2. Release of intact oligosaccharide chains with anhydrous hydrazine.
3. Treatment of the intact oligosaccharide chains with anhydrous methanolic HCl to liberate individual monosaccharides as O-methyl derivative.
4. N-acetylation of any primary amino groups.
5. Derivatization to give per-O-trimethylsilyl methyl glycosides.
6. Separation of these derivative, by capillary GLC (gas -liquid chromatography) on a CP-SIL8 column.
7. Identification of individual glycoside derivatives by retention time from the GLC and mass spectroscopy, compared to known standards.
8. Quantitation of individual derivatives by FID with an internal standard (13-O-methyl-D-glucose).

Neutral and amino-sugars can be determined by high performance anion-exchange chromatography combined with pulsed amperometric detection (HPAE-PAD Carbohydrate System, Dionex Corp.). For instance, sugars can be released by hydrolysis in 20% (v/v) trifluoroacetic acid at 100 C for 6 h. Hydrolysates are then dried by lyophilization or with a Speed-Vac (Savant Instruments). Residues are then dissolved in 1% sodium acetate trihydrate solution and analyzed on a HPLC-AS6 column as described by Anumula et al. (Anal. Biochem. 195:269-280 (1991).

Sialic acid can be determined separately by the direct colorimetric method of Yao et al. (Anal Biochem. 179: 332-335 (1989)) in triplicate samples. In a preferred embodiment the thiobarbaturic acid (TBA) of Warren, L. J. Biol Chem 238:(8) (1959) is used.

Alternatively, immunoblot carbohydrate analysis may be performed. According to this procedure protein-bound carbohydrates are detected using a commercial glycan detection system (Boehringer) which is based on the oxidative immunoblot procedure described by Haselbeck and Hosel [Haselbeck et al. Glycoconjugate J., 7:63 (1990)]. The staining protocol recommended by the manufacturer is followed except that the protein is transferred to a polyvinylidene difluoride membrane instead of nitrocellulose membrane and the blocking buffers contained 5% bovine serum albumin in 10 mM tris buffer, pH 7.4 with 0.9% sodium chloride. Detection is made with anti-digoxigenin antibodies linked with an alkaline phosphate conjugate (Boehringer), 1:1000 dilution in tris buffered saline using the phosphatase substrates, 4-nitroblue tetrazolium chloride, 0.03% (w/v) and 5-bromo-4 chloro-3-indoyl-phosphate 0.03% (w/v) in 100 mM tris buffer, pH 9.5, containing 100 mM sodium chloride and 50 mM magnesium chloride. The protein bands containing carbohydrate are usually visualized in about 10 to 15 min.

The carbohydrate may also be analyzed by digestion with peptide-N-glycosidase F. According to this procedure the residue is suspended in 14 1 of a buffer containing 0.18% SDS, 18 mM beta-mercaptoethanol, 90 mM phosphate, 3.6 mM EDTA, at pH 8.6, and heated at 100 EC for 3 min. After cooling to room temperature, the sample is divided into two equal parts. One aliquot is not treated further and serves as a control. The second fraction is adjusted to about 1% NP-40 detergent followed by 0.2 units of peptide-N-glycosidase F (Boehringer). Both samples are warmed at 37° C for 2 hr and then analyzed by SDS-polyacrylamide gel electrophoresis.

Preferred methods of analysis include those described for the analysis of antibody associated oligosaccharides and described in, for example Worald et al., (1997) Biochem. 36:1370-1380; Sheeley et al. (1997) Anal. Biochem. 247: 102-110 and Cant et al., (1994) Cytotechnology 15:223-228 as well as the references cited therein.

The recovered glycoproteins are purified according to known techniques employed in antibody preparations as described herein. The recovered purified antibodies are analyzed to confirm primary and secondary structure as described herein and in the Figures and Examples. Techniques for the analysis of intact glycoproteins are known in the art (Cant et al., (1994) Cytotechnology 15:223-228; Iwase et al., (1996) J. Biochem. 120:393-397; Sheeley et al., (1997) Analytical Biochemistry, 247:102-110). Typically the structural analysis is followed by functional analysis. As will be appreciated by the skilled artisan the constant domains are not involved directly in binding an antibody to an antigen, but exhibit various effector functions, such as participation of the antibody in antibody-dependent cellular toxicity and complement mediated cell lysis. The binding site on IgG for Clq, the first component of the complement cascade has been localized to the CH2 domains. Therefore standard analysis such as assays for complement dependent cytotoxicity such as those described for anti CD-20 antibodies are appropriate (Gazzano-Santoro et al., (1997) J. Immunol. Methods 202:163-171). Assays for antigen-mediated aggregation of IgG1, IgG2 and IgG3 initiates complement activation, binding

of IgG to the high affinity Fc receptors on monocytes which can stimulate those cells to eliminate the antigen to which the Ig is bound are appropriate for analyzing the functional activity of the recovered glycoprotein as well.

Therapeutic Compositions and Methods

Use of the glycoproteins of the present invention as therapeutic compositions is an embodiment of the invention. The uses generally disclosed herein are provided as guidance for the use of the preparations in general. The monoclonal antibody C2B8 (anti-CD20) is provided as an example of a monoclonal antibody developed for cancer treatment as noted above.

Therapeutic formulations of an antibody are prepared for storage by mixing the antibody having the desired degree of purity with optional physiologically acceptable carriers, excipients, or stabilizers (Remington's Pharmaceutical Sciences, 16th Edition, Osol., A., Ed., (1980)), in the form of lyophilized cake or aqueous solutions. Pharmaceutically acceptable carriers, excipients, or stabilizers are non-toxic to recipients at the dosages and concentrations employed, and include buffers such as phosphate, citrate, and other organic acids; antioxidants including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as Tween™, Pluronics™, or polyethylene glycol (PEG).

A antibody to be used for in vivo administration must be sterile. This is readily accomplished by filtration through sterile filtration membranes, prior to or following lyophilization and reconstitution. The formulation ordinarily will be stored in lyophilized form or in solution.

Therapeutic antibody compositions generally are placed into a container having a sterile access port, for example, an intravenous solution bag or vial having a stopper pierceable by a hypodermic injection needle.

The route of administration is in accord with known methods, e.g., injection or infusion by intravenous, intraperitoneal, intracerebral, intramuscular, intraocular, intraarterial, or intralesional routes, or by sustained-release systems as noted below. The antibody is administered continuously by infusion or by bolus injection.

A cancer patient to be treated with an antibody as an antagonist as disclosed herein may also receive radiation therapy. Alternatively, or in addition, a chemotherapeutic agent may be administered to the patient. Preparation and dosing schedules for such chemotherapeutic agents may be used according to manufacturers instructions or as determined empirically by the skilled practitioner. Preparation and dosing schedules for such chemotherapy are also described in Chemotherapy Service Ed., M.C. Perry, Williams & Wilkins, Baltimore, MD (1992). The chemotherapeutic agent may precede, or follow administration of the antagonist or may be given simultaneously therewith. For cancer indications, it may be desirable to also administer antibodies against tumor associated antigens or against angiogenic factors, such as antibodies which bind to HER2 or vascular endothelial factor (VEGF). Alternatively, or in addition, one or more cytokines may be co-administered to the patient.

An effective amount of antibody to be employed therapeutically will depend, for example, upon the therapeutic objectives, the route of administration, and the condition of the patient. Accordingly, it will be necessary for the therapist to titer the dosage and modify the route of administration as required to obtain the maximum therapeutic effect. A typical dosage might range from about 1 μg/kg to up to 100 mg/kg of patient body weight, preferably about 10 μg/kg to 10 mg/kg. Typically, the clinician will administer antagonist until a dosage is reached that achieves the desired effect for treatment of the above mentioned disorders. For C2B8 reference is made to International Publication No. WO 94/11026 and EP B 669836, the disclosures of which are specifically incorporated herein by reference.

Routes of administration for the individual or combined therapeutic compositions of the present invention include standard routes, such as, for example, intravenous infusion or bolus injection.

The invention further provides an article of manufacture and kit containing materials useful for the treatment of cancer, for example. The article of manufacture comprises a container with a label. Suitable containers include, for example, bottles, vials, and test tubes. The containers may be formed from a variety of materials such as glass or plastic. The container holds a composition comprising the glycoprotein preparations described herein. The active agent in the composition is the particular glycoprotein such as C2B8. The label on the container indicates that the composition is used for the treatment or prevention of a particular disease or disorder, and may also indicate directions for in vivo, such as those described above.

The kit of the invention comprises the container described above and a second container comprising a buffer. It may further include other materials desirable from a commercial and user standpoint, including other buffers, diluents, filters, needles, syringes, and package inserts with instructions for use.

The following examples are offered by way of illustration and not by way of limitation.

## EXAMPLES

### EXAMPLE I

Introduction

Substantially homogenous glycoprotein preparations are prepared with reference to the following Examples

Methods

The chimeric monoclonal anti-CD20 antibody (IDEC-C2B8) was produced and purified as described previously (Liu et al., (1987) J. Immunol. 139:3521; Maloney et al., (1994) Blood 84:2457). Other IgG molecules such as anti-HER2 (anti-P185$^{HER2}$ Carter et al., (1992) Proc. natl. Acad. Sci. USA 89:4285), anti-VEGF (Kim et al., (1992) Growth Factors 7:53-64), anti-IgE (Presta et al., (1993) J. Immunol. 151:2623) and TNFR-IgG (tumor necrosis factor receptor-IgG; Ashkenazi et al., (1991) Proc. Natl. Acad. Sci. USA 88:10535) were produced by recombinant DNA techniques and expressed in CHO cells. β1,4-galactosyltransferases from human and bovine sources were from Boehringer Mannheim (Indianapolis, IN) and Sigma Chemical Co. (St. Louis, Mo) respectively. UDP-Gal was obtained from Boehringer Mannheim (Indianapolis, IN). Penicillin, streptomycin, glutamine, HEPES, lyophilized rabbit serum and human serum used as the source of complement were purchased from GIBCO-BRL (Grand Island, NY). Fetal bovine serum was purchased from Hyclone Laboratories (Logan, UT). Bovine serum albumin (BSA) and Trypan blue were purchased from Sigma Chemical Co. (St. Louis, Mo). Alamar blue reagent was from Accumed International (Westlake, OH). NAP-5 and Protein A-Sepharose columns were purchased from Pharmacia (Sweden). Sodium cyanoborohydride in tetrahydrofuran was from Aldrich Chemical Co.

IDEC-C2B8

IDEC-C2B8 was formulated at 10 mg/ml in 25 mM sodium citrate, 150 mM sodium chloride, and 0.07 mg/mL Polysorbate 80 at pH 6.5.

EXAMPLE II

Enzyme digestion procedure: IgG samples (5-10 mg) were buffer-exchanged into 100 mM citrate-phosphate buffer, pH 5.0, using NAP-5 columns (Pharmacia). β-Galactosidase (40 mU/mg protein, *Diplococcus pneumoniae,* Boehringer Mannheim) and N-aceryl-β-D-glucosaminidase (40 mU/mg protein, *Diplococcus pneumoniae,* Boehringer Mannheim or jack bean enzyme from Sigma) were added to 5-10 mg aliquots of protein and incubated at 37°C for 12-24 hrs. The IgG samples were purified on a Protein A column. The purified protein was subjected to electrospray ionization mass spectrometry, capillary electrophoresis for carbohydrate analysis. The bioactivity was examined by CDC bioassay, binding assay and C1q binding studies.

Figure 1 A, Figure 1B and Figure 1C depict oligosaccharide analysis of an anti-CD20 monoclonal antibody C2B8 by capillary electrophoresis with laser-induced fluorescence detection. In Figure 1A C2B8 produced in 400L batch-fed culture produced at least three glycoforms of C2B8. Figure 1B depicts the same C2B8 preparation treated with galactosidase. Figure 1C depicts the preparation treated with both β-galactosidase and N-acerylglucosaminosidase A single G-2 glycoform preparation was obtained.

EXAMPLE III

C1q binding was assessed using the method of Reff et al., (1994) Blood 83:435-45. Briefly, 50μl of wil2 cells were mixed with various amounts of C2B8 to which various amounts of C1q were added. The cells were washed with buffer several times and the amount of C 1q bound was measured using florescently labeled anti-C1q antibody.

Figure 2 depicts the binding of C2B8 to C1q. Both G2 and G-2 preparations bound C1q to a greater extent than control samples exhibiting heterogeneous glycoforms.

Complement dependent cytotoxicity: The CDC bioassay of C2B8 samples was performed using RHBP (RPM1-1640 supplemented with 0.1% BSA, 20 mM HEPES (pH 7.2-7.4), 100 IU/ml penicillin and 100 μg/ml streptomycin. For the assay, 50 μl of $10^6$ cells/ml cell suspension, 50 μl of various concentrations of C2B8 and 50 ml of a 1/5 rabbit complement or human complement dilution were added to flat-bottomed 96-well tissue culture plates and incubated for 2 h at 37°C and 5% $CO_2$ to facilitate complement-mediated cell lysis. Fifty microliters of Alamar blue (undiluted, proprietary formulation of Accumed International) was then added and the incubation continued for another 5 h. The plates were allowed to cool to room temperature for 10 min on a shaker and the fluorescence was read using a 96-well fluorometer

with excitation at 530 nm and emission at 590 nm. Results are expressed in relative fluorescence units (RFU). RFU were plotted against C2B8 concentrations using a 4-parameter curve-fitting program (kaleidaGraph) and the sample concentrations were computed from the standard curve. All C2B8 concentrations shown throughout this report refer to final concentrations in the wells before the addition of Alamar blue (Gazzano-Santoro (1997) J. Immunol. Meth. 202: 163-171).

Figure 3 depicts the bioactivity of the G2 and G-2 glycoform preparation compared with the heterogenous composition for C2B8 in a rabbit complement lysis assay.

Example IV

**GALACTOSYLATION WITH GALACTOSYLTRANSFERASE:**

The antibody samples (IDEC-C2B8, anti-HER2, anti-VEGF, anti-IgE and TNFR-IgG), 10 mg in 0.5 ml, were buffer exchanged into 50 mM sodium cacodylate buffer, pH 7.1 (final vol. 1.0 ml). 50 μl each of 100 mM UDP-Gal and 100 mM $MnCl_2$ were added to the antibody solution. The β1,4-galactosyltransferase (b1,4GT; lyophilized powder) was reconstituted in 50 mM sodium cacodylate buffer pH 7.1 at a concentration of 1 mU/ml. 50 μl of this solution was added to the reaction mixture and incubated at 37°C for 48 hr. The reaction was stopped by cooling the reaction vial on ice (4°C) for 10 min. The galactosylated antibody was purified on protein A column.

Example V

**PURIFICATION OF GALACTOSYLATED ANTIBODY ON PROTEIN-A COLUMN:**

The reaction mixture containing galactosylated antibody was applied to a Protein A-Sepharose column (5 ml). The column was washed with at least 5 column volumes of phosphate buffered saline (pH 7.0) and the bound antibody was eluted with 100 mM citric acid, pH 3.0, which was immediately adjusted to pH 6.5 by adding 500 mM Tris-HCl buffer pH 8.0.

**<u>Analysis of the N-linked oligosaccharides</u>**

**Release and Labeling of N-linked oligosaccharides**

Protein samples (500-1000 μg) were buffer exchanged into 20 mM sodium phosphate buffer containing 50 mM EDTA and 0.02% (w/v) sodium azide, pH 7.5, using NAP-5 columns (Pharmacia). Five to ten units of recombinant peptide-N-glycosidase F (Oxford Glycosystems/Boehringer Mannheim) was added to the samples and incubated for 15 hours at 37°C. The deglycosylated protein was precipitated by heating at 95°C for 5 minutes and removed by centrifugation at 10,000 g for 10 minutes. The supernatant containing the released oligosaccharides was dried in a centrifugal vacuum evaporator and labeled by the addition of 15 μL of 1.9 mM solution of 9-aminopyrene-1,4,6-trisulfonate (APTS, Beckmann) in 15% acetic acid and 5 μL of 1 M sodium cyanoborohydride in tetrahydrofuran. The labeling reaction was carried out for 2 hours at 55°C, diluted in water (0.5 ml) and analyzed by capillary electrophoresis (CE).

Example VI

**Capillary Electrophoresis Analysis**

CE analysis of the labeled oligosaccharides was performed on a P/ACE 5000 CE system (Beckman) with the polarity reversed, using a coated capillary of 50 mm internal diameter and 20 cm effective length (eCAP, N-CHO coated capillary, Beckmann). The samples were introduced by pressure injection at 0.5 psi. for 8 seconds and electrophoresis was carried out at a constant voltage of 740 V/cm. The temperature of the capillary was maintained at 20°C. The separations were monitored on-column with a Beckmann laser-induced fluorescence detection system using a 3 mW argon-ion laser with an excitation wavelength of 488 nm and emission bandpass filter at 520 x 10 nm.

Results

Figure 4A and Figure 4B depict oligosaccharide analysis of an anti-CD20 monoclonal antibody C2B8 by capillary electrophoresis with laser-induced fluorescence detection. In Figure 4A C2B8 produced in 400L batch-fed culture produced at least three glycoforms of C2B8. Figure 4B depicts the same C2B8 preparation treated with β1-4 galactosyltransferase according the present invention. A single G2 glycoform preparation was obtained.

Figure 5 depicts analysis of an anti-VEGF monoclonal antibody by capillary electrophoresis. In Figure 5 anti-VEGF produced in CHO cell culture produced at least three glycoforms forming a heterogenous composition. The same anti-VEGF treated with β-1-4 galactosyltransferase according the present invention produced a single G2 glycoform.

Figure 6 depicts analysis of an anti-IgE monoclonal antibody by capillary electrophoresis. In Figure 6 anti-IgE produced in CHO cell culture produced at least three glycoforms forming a heterogenous oligosaccharide population. The same anti-IgE CHO cell composition treated with β-1-4 galactosyltransferase according the present invention produced a single G2 glycoform.

Figure 7 depicts analysis of an anti-HER2 monoclonal antibody by capillary electrophoresis. In Figure 7 anti-HER2 produced in CHO cell culture produced at least three glycoforms forming a heterogenous oligosaccharide population. The same anti-HER2 CHO composition treated with β-1-4 galactosyltransferase according the present invention produced a single G2 glycoform.

Example V

**SODIUM DODECYLSULFATE POLYACRYLAMIDE GEL ELECTROPHORESIS (SDS-PAGE)**

Protein samples were diluted to 1.0 mg/mL into phosphate-buffered saline (PBS). The samples were diluted to 0.2 mg/mL for the silver stained gels and to 0.5 mg/mL for the immunoblots into sample buffer and heated for 3 minutes at 90°C. For reduced samples, the sample buffer contained 80 mM DTT. Samples (10 μL) were loaded onto Integrated Separation Systems (ISS) MiniPlus Sepragels, with a 4-20% acrylamide gradient. Electrophoresis was performed using the ISS Mini 2 gel apparatus at 30 mA per gel for 60 minutes. Novex Mark12 molecular weight standards were used in the silver stained gels whereas Amersham Rainbow molecular weight standards were used in the gels prepared for immunoblotting.

Silver Stain

SDS PAGE gels were incubated overnight in a fixing solution (40% ethanol, 10% acetic acid), washed in water and incubated in incubation solution (30% ethanol, 25% glutaraldehyde, 0.5 M sodium acetate, 10 mM sodium thiosulfate). The gels were washed again and incubated for 40 minutes in silver nitrate solution (6 mM silver nitrate, 0.01% formaldehyde), washed and developed with two changes of developing solution (0.3 M sodium carbonate, 0.01% formaldehyde). The reaction was stopped by incubating for 10 min in stop solution (40 mM EDTA) and then washed before scanning.

Figure 8 depicts a representative SDS polyacrylamide gel analysis of an anti-CD20 monoclonal antibody under non-reducing conditions. Lane 1 is molecular weight standards, Lane 2 is the G2 glycoform of C2B8; Lane 3 is the C2B8 preparation treated with galactosidase to remove galactose residues from the oligosaccharides; Lane 4 is the CHO derived C2B8 preparation treated with PNGase-F for the removal of intact oligosaccharide; Lane 5 is the C2B8 antibody from CHO production; Lane 6 is the CHO derived C2B8 after incubation at 37 C for 24 hours; lane 7 is the CHO derived C2B8 and BSA. The representative gel shows that the integrity of the C2B8 molecule remains intact after treatment with the galactosyltransferase. The G2 glycoform does not disrupt the primary structure of the antibody.

Figure 9 depicts the same material described above analyzed by polyacrylamide gel electrophoresis under reducing conditions. The C2B8 heavy and light chains remain intact.

Example VI

**ELECTROTRANSFER AND IMMUNOSTAINING**

After SDS-PAGE, the protein was electrotransferred to nitrocellulose (0.2 m, Scleicher and Schuell) in a NovaBlot Semi-Dry electrotransfer apparatus in transfer buffer (39 mM glycine, 48 mM TRIS, 0.04% SDS, 20% methanol) for 90 minutes at 10 V. After electrotransfer, the nitrocellulose sheets were blocked in gelatin buffer (50 mM TRIS, 150 mM NaCl, 4.3 mM EDTA, 0.05% Triton X-100, and 0.25% fish gelatin). The immunoblots were probed with an affinity purified goat anti-human IgG (Jackson Laboratories) or goat anti-CHOP (IDEC Pharmaceuticals). Following incubation with the primary antisera the nitrocellulose sheets were washed with gelatin buffer and then incubated for 90 minutes with a rabbit anti-goat 1gG-HRP (Jackson-Immunoresearch). The immunoblots were washed with gelatin buffer and then PBS/Tween 20. The immunoblots were stained with the substrate solution; 3,3'-diaminobenzidine tetrahydrochloride dihydrate (DAB), 0.5 mg/mL, nickel ammonium sulfate, 0.3 mg/mL; cobalt chloride, 0.3 mg/mL in PBS with $H_2O_2$.

Example VII

**CIRCULAR DICHROISM SPECTROSCOPY**

The circular dichroic (CD) spectra of GT treated and untreated C2B8 was obtained on an AVIV 60DS spectropolarimeter. Each sample was dialyzed against 25 mM sodium citrate and 150 mM sodium chloride and then pipetted into a 0.01-cm thermostatted circular cuvette. Each spectrum was the sum of 5 scans from 200 to 250 nm. The spectra were obtained at 20°C. The protein concentration was determined using a $A^{0.1\%} = 1.7\ cm^{-1}$ at 280 nm. The mean residue weight ellipticity was calculated from

$$[Q]_{MRW} = Q_{obs}*(MRW)/\ 10\ c\ l$$

where $Q_{obs}$ is the ellipticity of the sample, MRW is the mean residue weight of the IDEC-C2B8 (108.8), c is the sample concentration in mg/mL, and 1 is the path length of the cell in cm. The content of the secondary structural elements, α-helix, β-sheet, and non-ordered structure was calculated using the program CONTIN (Provencer and Glockner (1981) Biochem. 20:33-37; Provencer (1982) Comput. Phys. Commun. 27:229-242).

Figure 10A and Figure 10B depict far and near UV CD spectra of C2B8 antibody from CHO culture and the G2 glycoform. As can be concluded from this analysis the G2 glycoform examined by circular dichroism as an indication of secondary structure (Provencer and Glockner (1981), Biochem. 20:33-37) is the same as the heterogenous C2B8 composition.

Example VIII

**CULTURE OF WIL2-S CELLS:**

The human B-lymphoblastoid cell line WIL2-S was obtained from the American Type Culture Collection (ATCC, Rockville, MD). The cells were grown in RPMI-1640 medium supplemented with 10% heat-inactivated (56°C, 30 min) fetal bovine serum, 2 mM glutamine and 20 mM HEPES, pH 7.2. Cells were cultured at 37°C in a humidified 5% $CO_2$ incubator.

**COMPLEMENT-DEPENDENT CYTOTOXICITY BIOASSAY:**

The CDC bioassay of C2B8 samples was performed using RHBP (RPMI-1640 supplemented with 0.1% BSA, 20 mM HEPES (pH 7.2-7.4), 100 IU/ml penicillin and 100 μg/ml streptomycin. For the assay, 50 μl of $10^6$ cells/ml cell suspension, 50 μl of various concentrations of C2B8 and 50 ml of a 1/5 rabbit complement or human complement dilution were added to flat-bottomed 96-well tissue culture plates and incubated for 2 h at 37°C and 5% $CO_2$ to facilitate complement-mediated cell lysis. Fifty microliters of Alamar blue (undiluted, proprietary formulation of Accumed International) was then added and the incubation continued for another 5 h. The plates were allowed to cool to room temperature for 10 min on a shaker and the fluorescence was read using a 96-well fluorometer with excitation at 530 nm and emission at 590 nm. Results are expressed in relative fluorescence units (RFU). RFU were plotted against C2B8 concentrations using a 4-parameter curve-fitting program (kaleidaGraph) and the sample concentrations were computed fro the standard curve. All C2B8 concentrations shown throughout this report refer to final concentrations in the wells before the addition of Alamar blue (Gazzano-Santoro (1997) J. Immunol. Meth. 202:163-171).

Figure 11 depicts the bioactivity of the G2 glycoform preparation compared with the heterogenous composition for C2B8 in a rabbit complement lysis assay.

Figure 12 depicts the correlation of bioactivity and galactose content in the G2 glycoform. The G2 glycoform preparation was at least 1.5 times more active in this assay than that produced under typical cell culture conditions.

**Claims**

1. A composition comprising glycoprotein wherein at least one glycoprotein is a glycoprotein having at least one immunoglobulin heavy chain CH2 domain containing N-linked G-2 oligosaccharide and the amount of glycoprotein having an N-linked G1, G0, or G-1 oligosaccharide in the CH2 domain does not exceed 10% by weight.

2. The composition of claim 1 wherein the heavy chain CH2 domain contains N-linked G2 and G-2 oligosaccharide.

**3.** The composition of claim 1 or 2 wherein the N-linked G2 or G-2 oligosaccharide further comprises a bisecting N-acetylglucosamine.

**4.** The composition of any one of claims 1 to 3 wherein the glycoprotein is an antibody.

**5.** The composition of claim 4 wherein the glycoprotein is a monoclonal antibody.

**6.** The composition of claim 4 or 5 wherein the antibody is an IgG.

**7.** The composition of claim 6 wherein the IgG is human IgG1.

**8.** The composition of any one of claims 5 to 7 wherein the antibody is selected from the group consisting of an anti-CD20 specific monoclonal antibody, an anti-HER2 specific monoclonal antibody, an anti-VEGF specific monoclonal antibody, and an anti-IgE specific monoclonal antibody.

**9.** The composition of claim 8 wherein the monoclonal antibody is an anti-CD20 antibody.

**10.** The composition of any one of claims 1 to 3 wherein the glycoprotein is an immunoadhesin glycoprotein.

**11.** The composition of any one of claims 1 to 3 wherein the glycoprotein is an antibody-immunoadhesin chimera.

**12.** The composition of claim 11 wherein the immunoadhesin glycoprotein is a tumor necrosis factor-immunoglobulin G1 chimera.

**13.** The composition of any preceding claim wherein the amount of said glycoprotein having an N-linked G2 oligosaccharide in the CH2 domain does not exceed 10% by weight.

**14.** A method of producing the composition of any preceding claim comprising the steps of reacting in an aqueous buffered solution at a temperature of 25-40°C;

a) a β-galactosidase;
b) an N-acetyl-β-D-glucosaminidase;
c) a substrate glycoprotein; and recovering the glycoprotein.

**15.** The method of claim 14 wherein the β-galactosidase is from *Diplococcus pneumoniae*.

**16.** The method of claim 14 or claim 15 wherein the N-acetyl-β-D-glucosaminidase is from *Diplococcus pneumoniae* or jack bean.

**17.** The method of claim 15 or claim 16 wherein the reaction temperature is 37°C.

**18.** The composition of any one of claims 1 to 13 for use in a method of medical treatment.

**19.** A pharmaceutical composition comprising the composition of any one of claims 1 to 13 and a pharmaceutically acceptable carrier.

**20.** An article of manufacture, comprising:

a container;
a label on said container; and
the composition of any one of claims 1 to 13 contained within said container.

**21.** The article of claim 20 wherein the label on the container indicates that the composition can be used for the treatment of cancer.

## Patentansprüche

1. Zusammensetzung, umfassend Glykoprotein, worin zumindest ein Glykoprotein ein Glykoprotein mit zumindest einer Immunglobulinschwerketten-CH2-Domäne ist, die N-gebundenes G-2-Oligosaccharid enthält, und die Menge an Glykoprotein mit N-gebundenem G1-, G0- oder G-1-Oligosaccharid in der CH2-Domäne nicht mehr als 10 Gew.-% beträgt.

2. Zusammensetzung nach Anspruch 1, worin die Schwerketten-CH2-Domäne N-gebundenes G2- und G-2-Oligosaccharid enthält.

3. Zusammensetzung nach Anspruch 1 oder 2, worin das N-gebundene G2- oder G-2-Oligosaccharid ferner ein zweiteilendes N-Acetylglucosamin umfasst.

4. Zusammensetzung nach einem der Ansprüche 1 bis 3, worin das Glykoprotein ein Antikörper ist.

5. Zusammensetzung nach Anspruch 4, worin das Glykoprotein ein monoklonaler Antikörper ist.

6. Zusammensetzung nach Anspruch 4 oder 5, worin der Antikörper ein IgG ist.

7. Zusammensetzung nach Anspruch 6, worin das IgG menschliches IgG1 ist.

8. Zusammensetzung nach einem der Ansprüche 5 bis 7, worin der Antikörper aus der aus einem anti-CD20-spezifischen monoklonalen Antikörper, einem anti-HER2-spezifischen monoklonalen Antikörper, einem anti-VEGF-spezifischen monoklonalen Antikörper und einem anti-IgE-spezifischen monoklonalen Antikörper bestehenden Gruppe ausgewählt ist.

9. Zusammensetzung nach Anspruch 8, worin der monoklonale Antikörper ein Anti-CD20-Antikörper ist.

10. Zusammensetzung nach einem der Ansprüche 1 bis 3, worin das Glykoprotein ein Immunoadhäsinglykoprotein ist.

11. Zusammensetzung nach einem der Ansprüche 1 bis 3, worin das Glykoprotein eine Antikörper-Immunoadhäsin-Chimäre ist.

12. Zusammensetzung nach Anspruch 11, worin das Immunoadhäsinglykoprotein eine Tumornekrosefaktor-Immunglobulin-G1-Chimäre ist.

13. Zusammensetzung nach einem der vorangegangenen Ansprüche, worin die Menge des Glykoproteins mit einem N-gebundenen G2-Oligosaccharid in der CH2-Domäne nicht mehr als 10 Gew.-% beträgt.

14. Verfahren zur Herstellung der Zusammensetzung nach einem der vorangegangenen Ansprüche, umfassend die Schritte des Umsetzens von

    a) einer β-Galactosidase,
    b) einer N-Acetyl-β-D-glucosaminidase,
    c) einem Substratglykoprotein

    in einer wässrigen gepufferten Lösung bei einer Temperatur von 25 bis 40 °C und der Gewinnung des Glykoproteins.

15. Verfahren nach Anspruch 14, worin die β-Galactosidase von Diplococcus pneumoniae stammt.

16. Verfahren nach Anspruch 14 oder 15, worin die N-Acetyl-β-D-glucosaminidase von Diplococcus pneumoniae oder der Schwertbohne stammt.

17. Verfahren nach Anspruch 15 oder 16, worin die Reaktionstemperatur 37 °C beträgt.

18. Zusammensetzung nach einem der Ansprüche 1 bis 13 zur Verwendung in einem medizinischen Behandlungsverfahren.

**19.** Pharmazeutische Zusammensetzung, umfassend die Zusammensetzung nach einem der Ansprüche 1 bis 13 und einen pharmazeutisch annehmbaren Träger.

**20.** Fertigartikel, der Folgendes umfasst:

einen Behälter,
ein Etikett auf dem Behälter und
die Zusammensetzung nach einem der Ansprüche 1 bis 13, die in dem Behälter enthalten ist.

**21.** Artikel nach Anspruch 20, wobei das Etikett auf dem Behälter angibt, dass die Zusammensetzung zur Behandlung von Krebs eingesetzt werden kann.

**Revendications**

**1.** Composition comprenant une glycoprotéine, dans laquelle au moins une glycoprotéine est une glycoprotéine ayant au moins un domaine CH2 de chaîne lourde d'immunoglobuline contenant un oligosaccharide G-2 lié par N et la quantité de glycoprotéine ayant un oligosaccharide G1, G0 ou G-1 lié par N dans le domaine CH2 n'excède pas 10 % en poids.

**2.** Composition suivant la revendication 1, dans laquelle le domaine CH2 de chaîne lourde contient un oligosaccharide G2 et G-2 lié par N.

**3.** Composition suivant la revendication 1 ou 2, dans laquelle l'oligosaccharide G2 ou G-2 lié par N comprend en outre une N-acétylglucosamine de division en 2.

**4.** Composition suivant l'une quelconque des revendications 1 à 3, dans laquelle la glycoprotéine est un anticorps.

**5.** Composition suivant la revendication 4, dans laquelle la glycoprotéine est un anticorps monoclonal.

**6.** Composition suivant la revendication 4 ou 5, dans laquelle l'anticorps est une IgG.

**7.** Composition suivant la revendication 6, dans laquelle l'IgG est une IgG1 humaine.

**8.** Composition suivant l'une quelconque des revendications 5 à 7, dans laquelle l'anticorps est choisi dans le groupe consistant en un anticorps monoclonal anti-CD20 spécifique, un anticorps monoclonal anti-HER2 spécifique, un anticorps monoclonal anti-VEGF spécifique et un anticorps monoclonal anti-IgE spécifique.

**9.** Composition suivant la revendication 8, dans laquelle l'anticorps monoclonal est un anticorps anti-CD20.

**10.** Composition suivant l'une quelconque des revendications 1 à 3, dans laquelle la glycoprotéine est une glycoprotéine du type immunoadhésine.

**11.** Composition suivant l'une quelconque des revendications 1 à 3, dans laquelle la glycoprotéine est une chimère anticorps-immunoadhésine.

**12.** Composition suivant la revendication 11, dans laquelle la glycoprotéine du type immunoadhésine est une chimère facteur de nécrose tumorale-immunoglobuline G1.

**13.** Composition suivant l'une quelconque des revendications précédentes, dans laquelle la quantité de ladite glycoprotéine ayant un oligosaccharide G2 lié par N dans le domaine CH2 n'excède pas 10 % en poids.

**14.** Procédé pour la production de la composition de l'une quelconque des revendications précédentes, comprenant les étapes de réaction dans une solution aqueuse tamponnée, à une température de 25 à 40°C ;

a) d'une β-galactosidase ;
b) d'une N-acétyl-β-D-glucosaminidase ;
c) d'une glycoprotéine servant de substrat ; et de récupération de la glycoprotéine.

**15.** Procédé suivant la revendication 14, dans lequel la β-galactosidase provient de *Diplococcus pneumoniae*.

**16.** Procédé suivant la revendication 14 ou la revendication 15, dans lequel la N-acétyl-β-D-glucosaminidase provient de *Diplococcus pneumoniae* ou bien du haricot sabre.

**17.** Procédé suivant la revendication 15 ou la revendication 16, dans lequel la température réactionnelle est égale à 37°C.

**18.** Composition suivant l'une quelconque des revendications 1 à 13, destinée à être utilisée dans une méthode de traitement médical.

**19.** Composition pharmaceutique comprenant la composition de l'une quelconque des revendications 1 à 13 et un support pharmaceutiquement acceptable.

**20.** Article manufacturé, comprenant :

un récipient ;
une étiquette sur ledit récipient ; et
la composition de l'une quelconque des revendications 1 à 13 présente dans ledit récipient.

**21.** Article suivant la revendication 20, dans lequel l'étiquette sur le récipient indique que la composition peut être utilisée pour le traitement du cancer.

Before enzyme treatment.

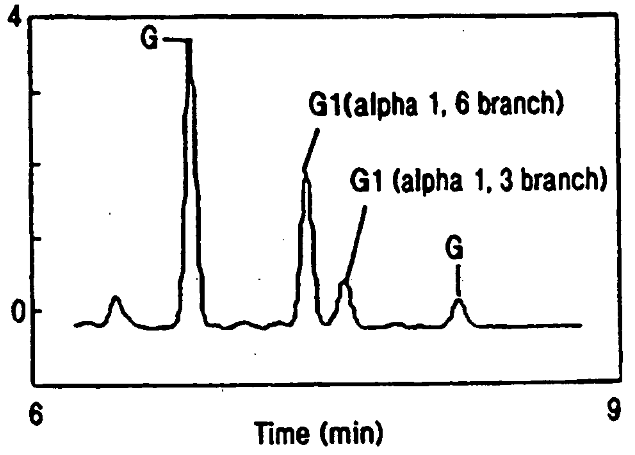


FIG. 1A

After treatment with ß-galactosidase.

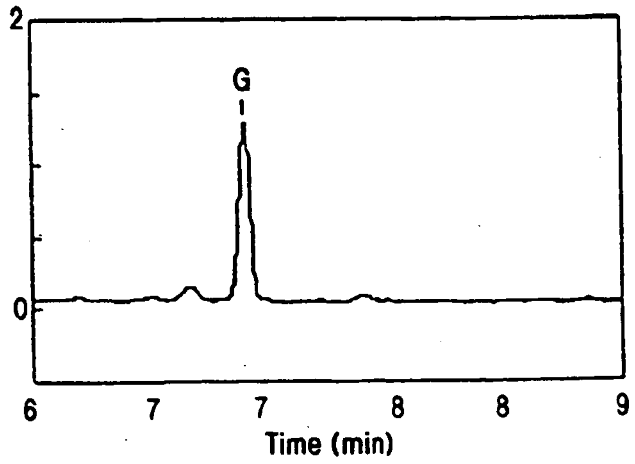


FIG. 1B

After treatment with ß-galactosidase and N-acetyl ß-D-glucosaminidase.

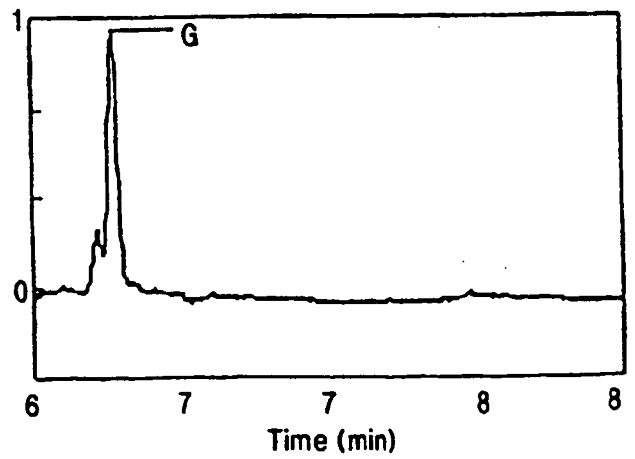


FIG. 1C

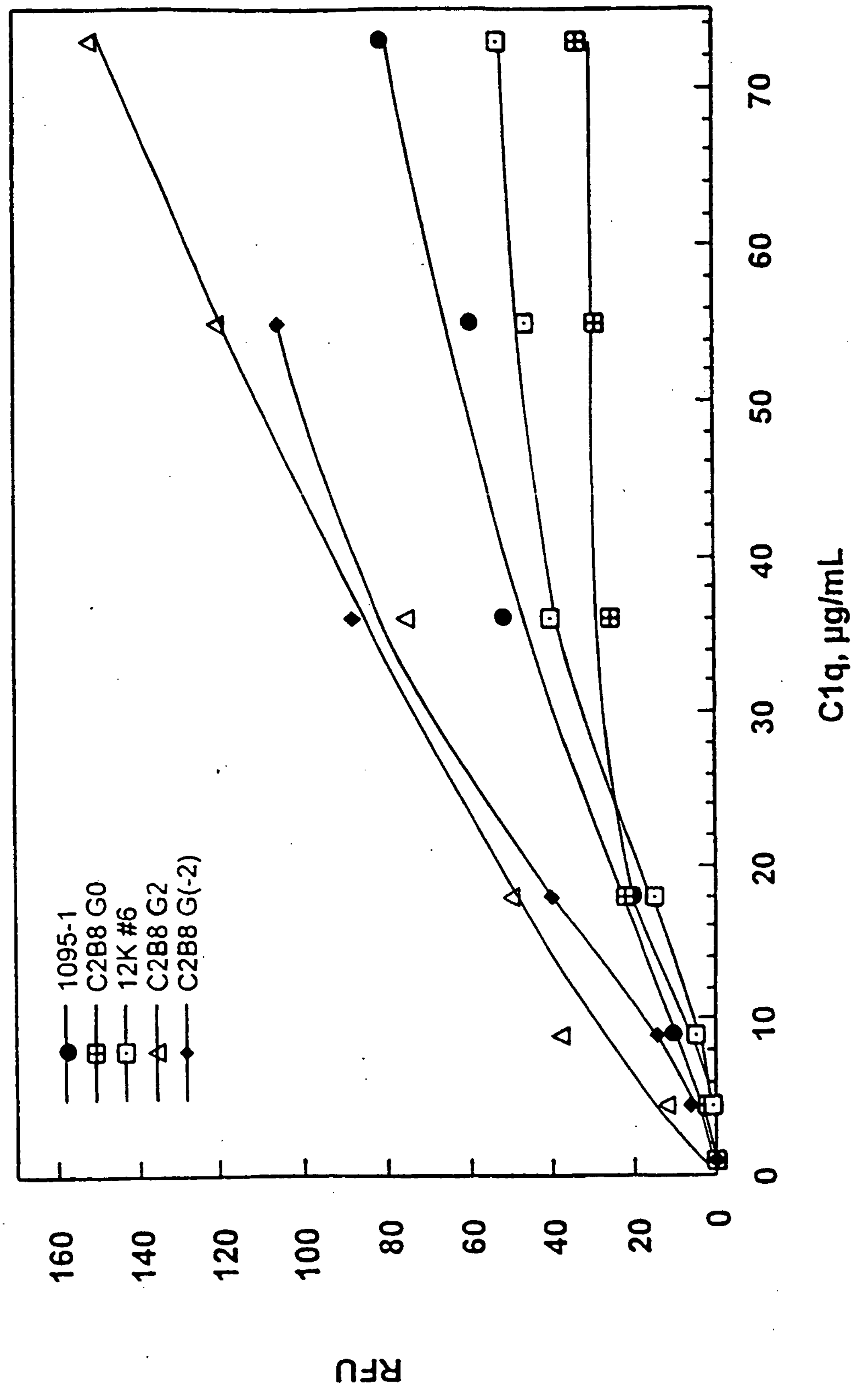
1095-1
C2B8 G0
12K #6
C2B8 G2
C2B8 G(-2)
RFU
0
20
40
60
80
100
120
140
160
0
10
20
30
40
50
60
70
C1q, µg/mL

FIG. 2

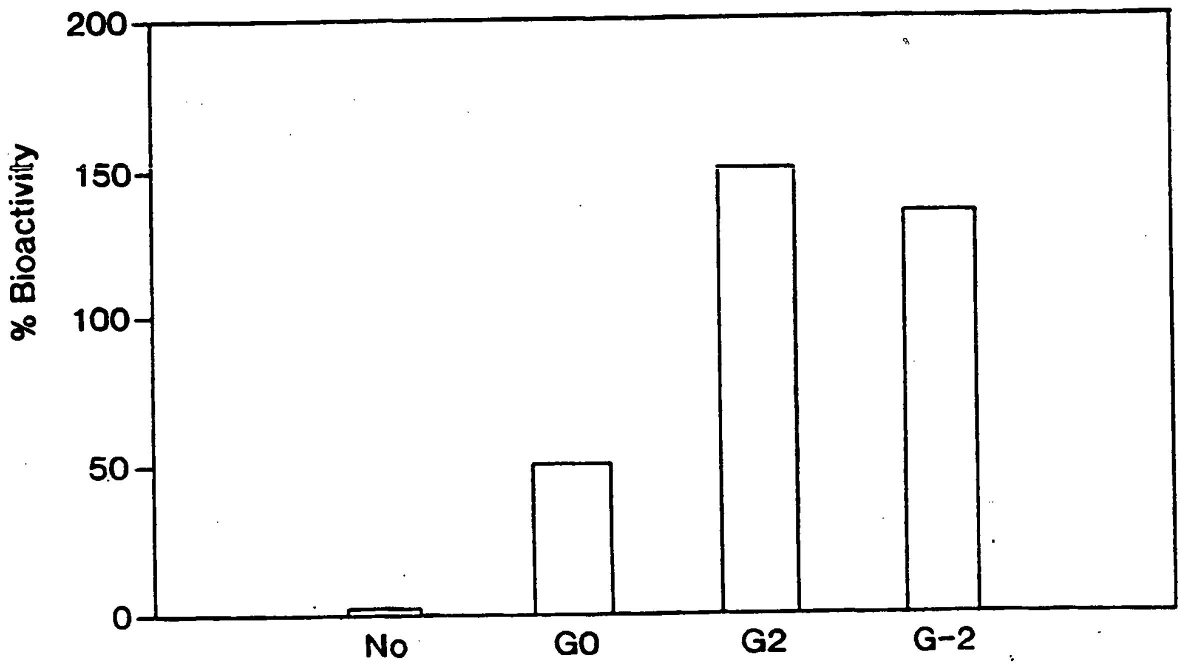


FIG. 3

FIG. 4A

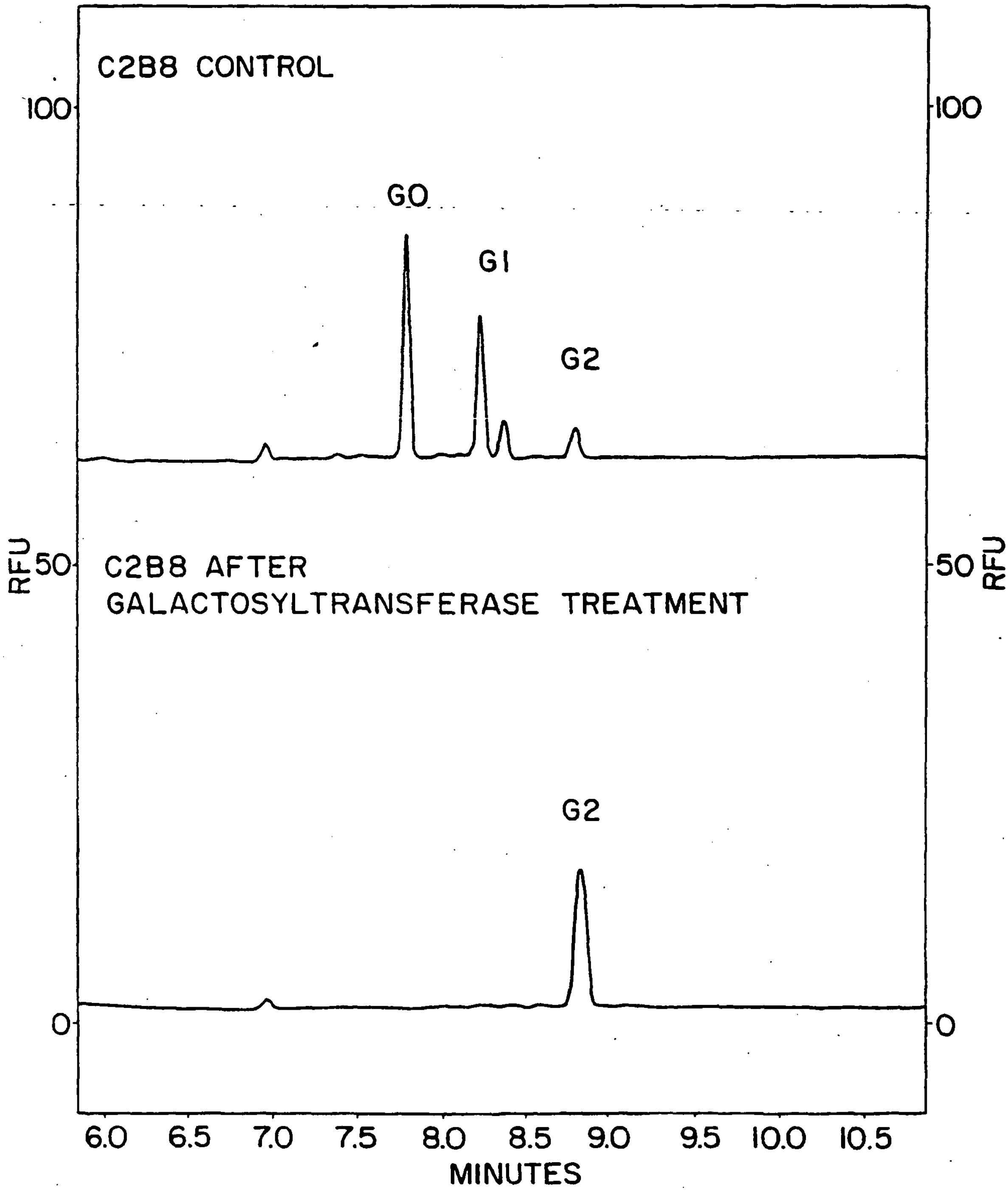
C2B8 CONTROL
100
100
GO
GI
G2
RFU
50
50
RFU
C2B8 AFTER
GALACTOSYLTRANSFERASE TREATMENT
G2
0
0
6.0
6.5
7.0
7.5
8.0
8.5
9.0
9.5
10.0
10.5
MINUTES

FIG. 4B

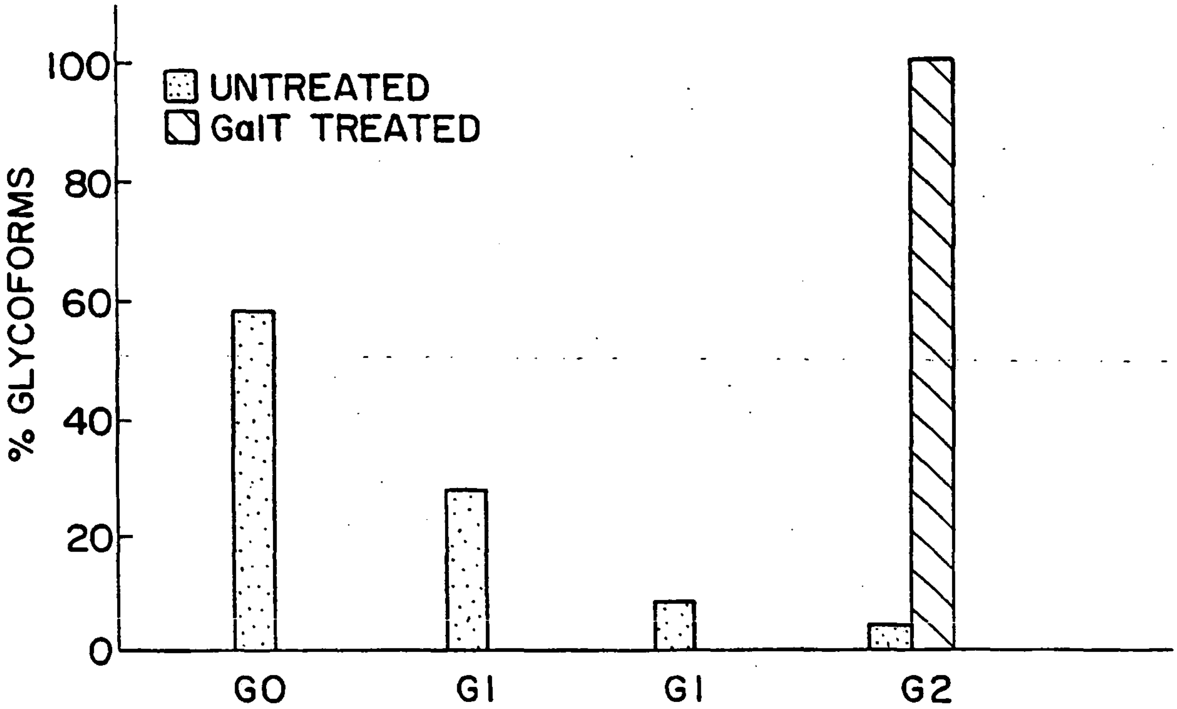

100
80
60
40
20
0
% GLYCOFORMS
UNTREATED
GalT TREATED
GO
G1
G1
G2


FIG. 5

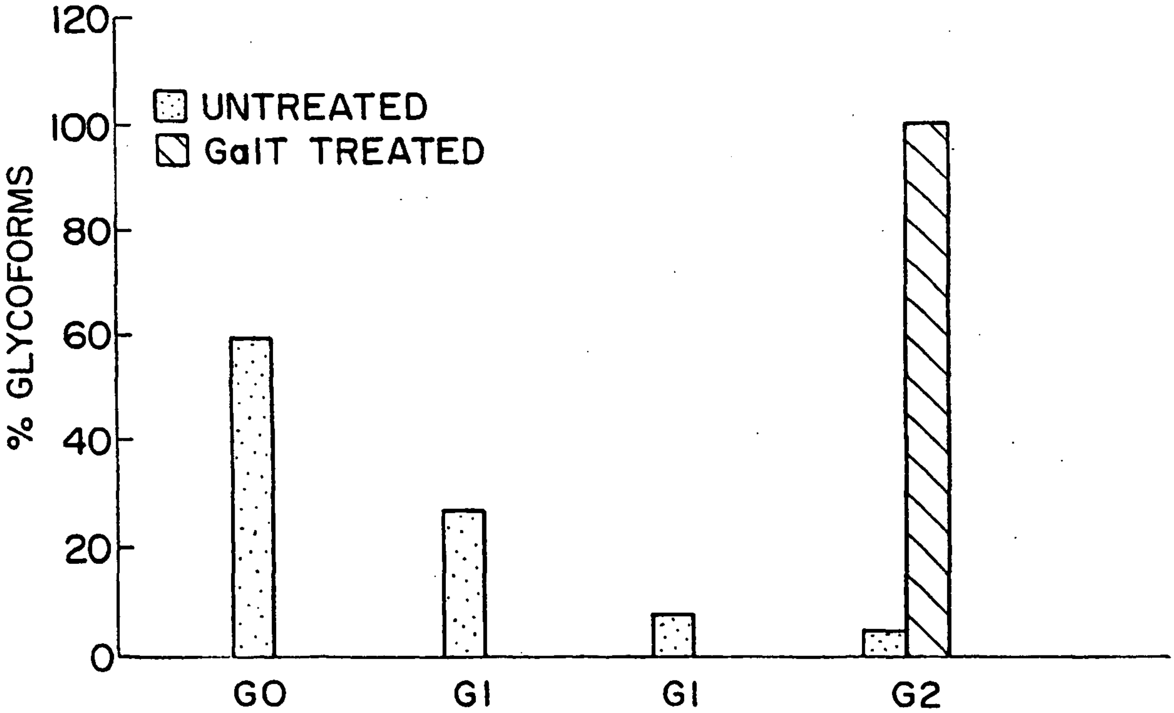

120
100
80
60
40
20
0
% GLYCOFORMS
UNTREATED
GalT TREATED
GO
G1
G1
G2


FIG. 6

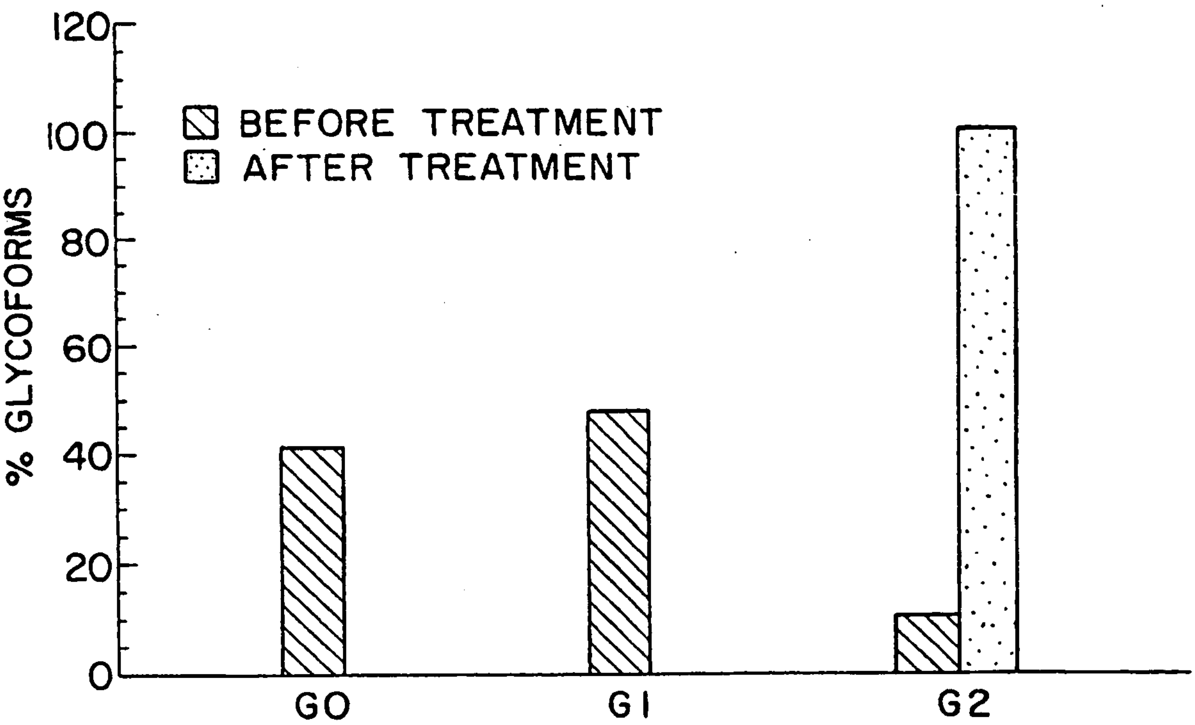
120
100
80
60
40
20
0
% GLYCOFORMS
BEFORE TREATMENT
AFTER TREATMENT
GO
G1
G2

FIG. 7

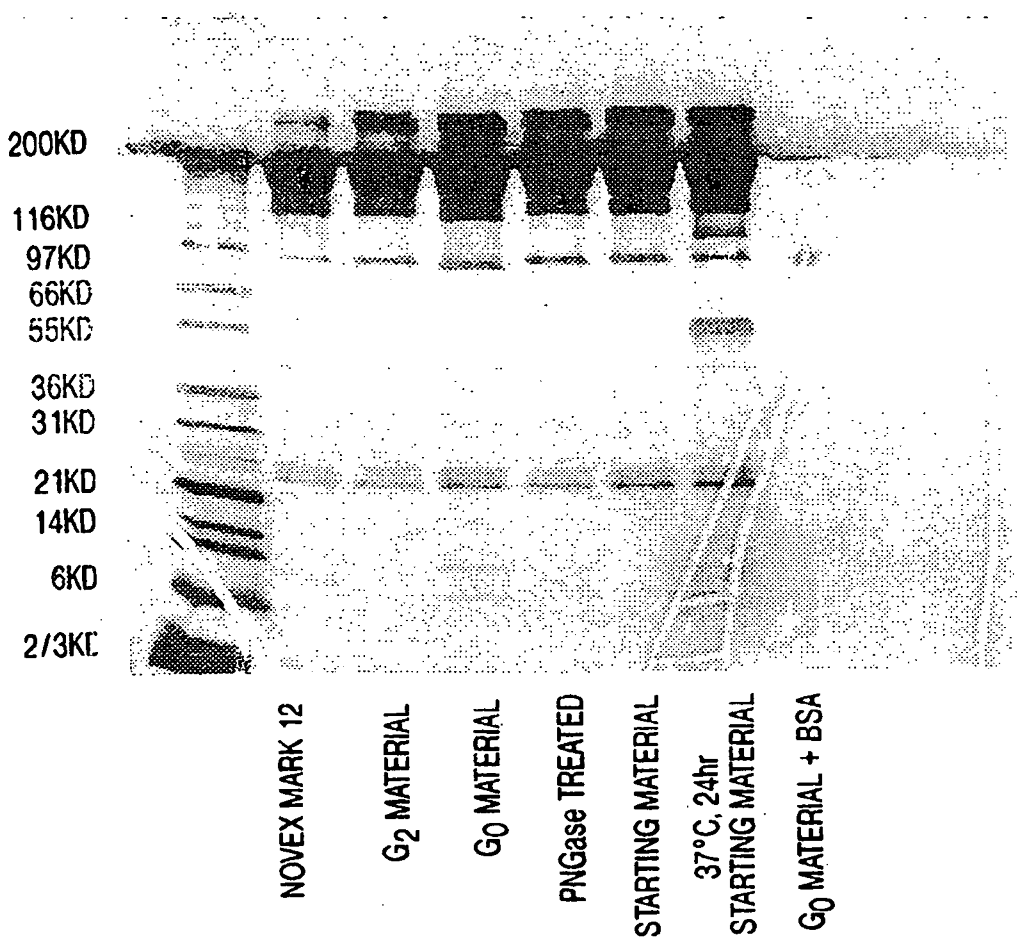
200KD
116KD
97KD
66KD
55KD
36KD
31KD
21KD
14KD
6KD
2/3KD
NOVEX MARK 12
G2 MATERIAL
G0 MATERIAL
PNGase TREATED
STARTING MATERIAL
37°C, 24hr
STARTING MATERIAL
G0 MATERIAL + BSA

FIG. 8

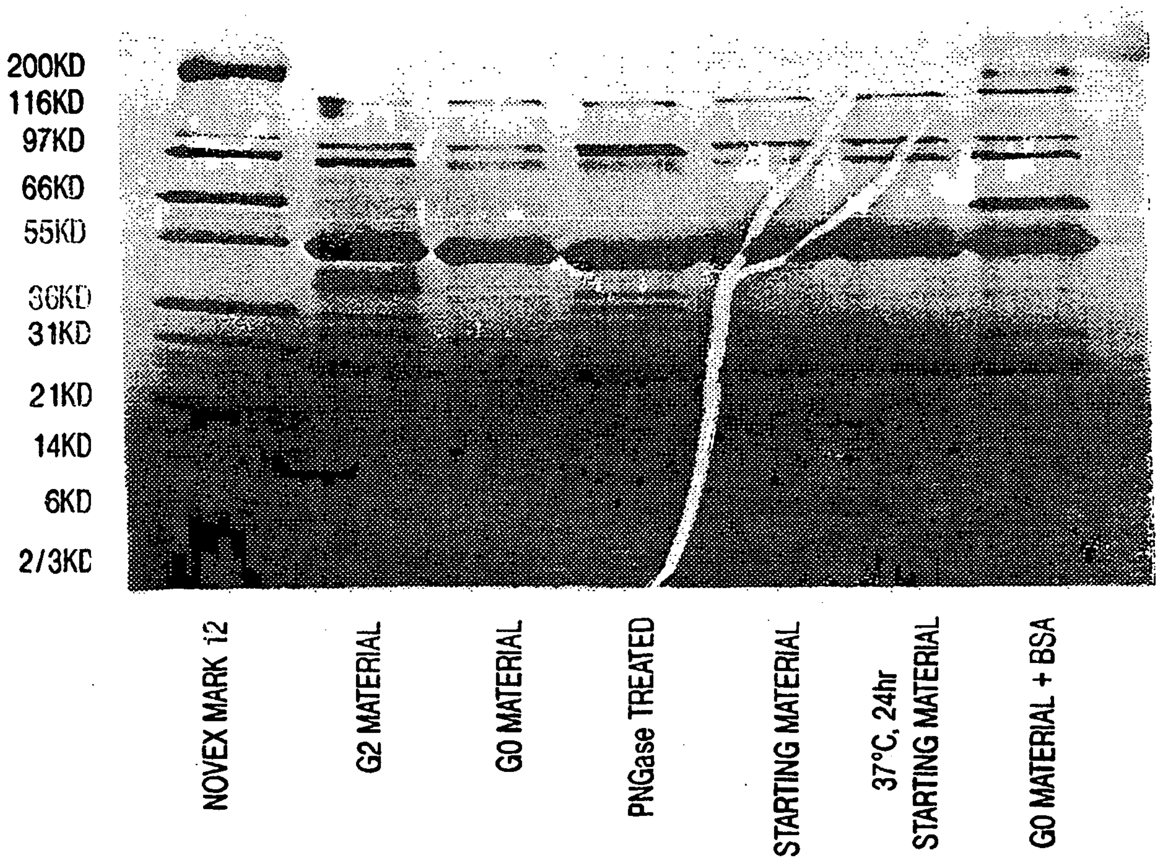
200KD
116KD
97KD
66KD
55KD
36KD
31KD
21KD
14KD
6KD
2/3KD
NOVEX MARK 12
G2 MATERIAL
G0 MATERIAL
PNGase TREATED
STARTING MATERIAL
37°C, 24hr STARTING MATERIAL
G0 MATERIAL + BSA

FIG. 9

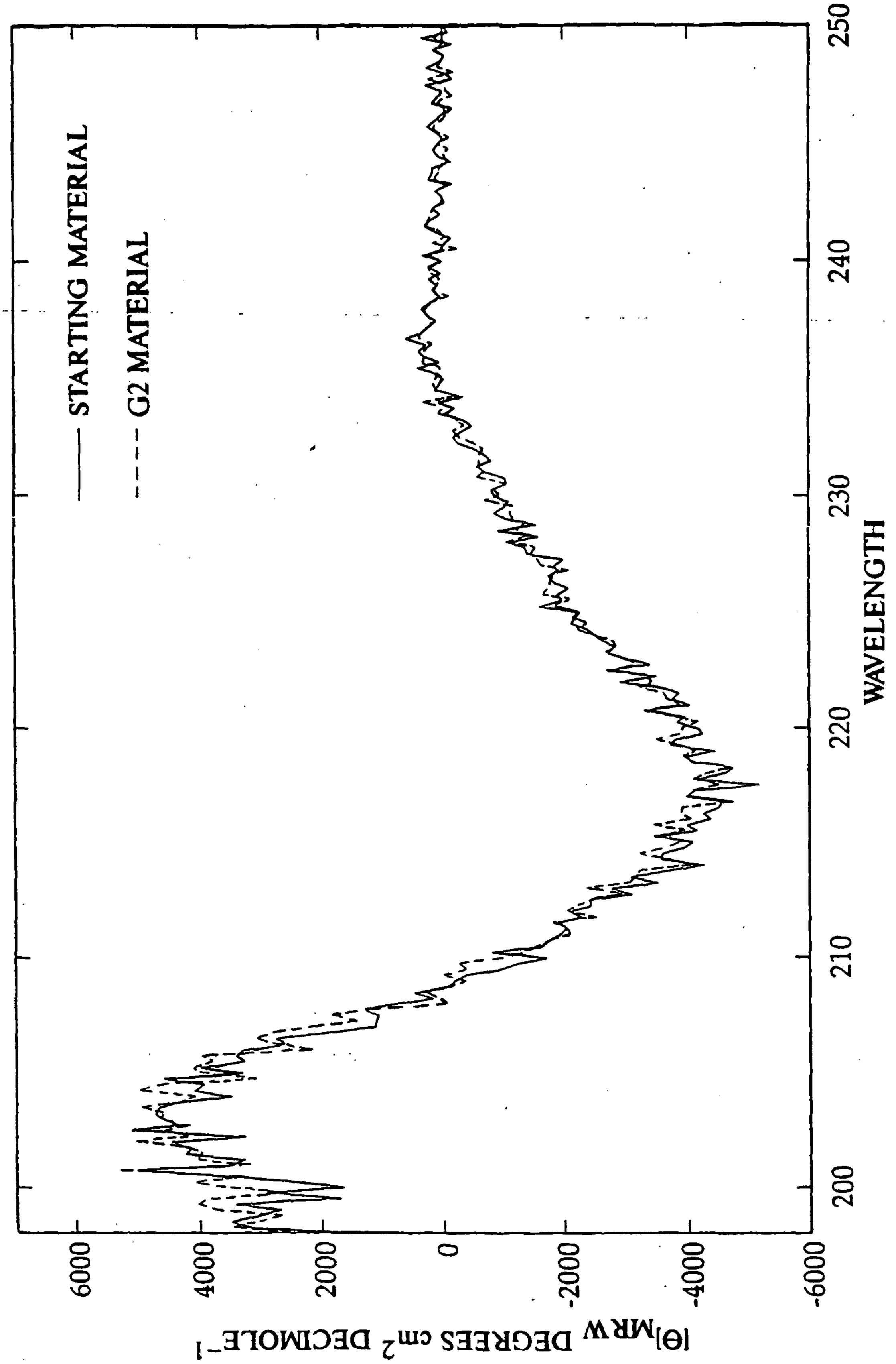
STARTING MATERIAL
G2 MATERIAL
WAVELENGTH
200
210
220
230
240
250
6000
4000
2000
0
-2000
-4000
-6000
[Θ]MRW DEGREES cm2 DECIMOLE-1

FIG. 10A

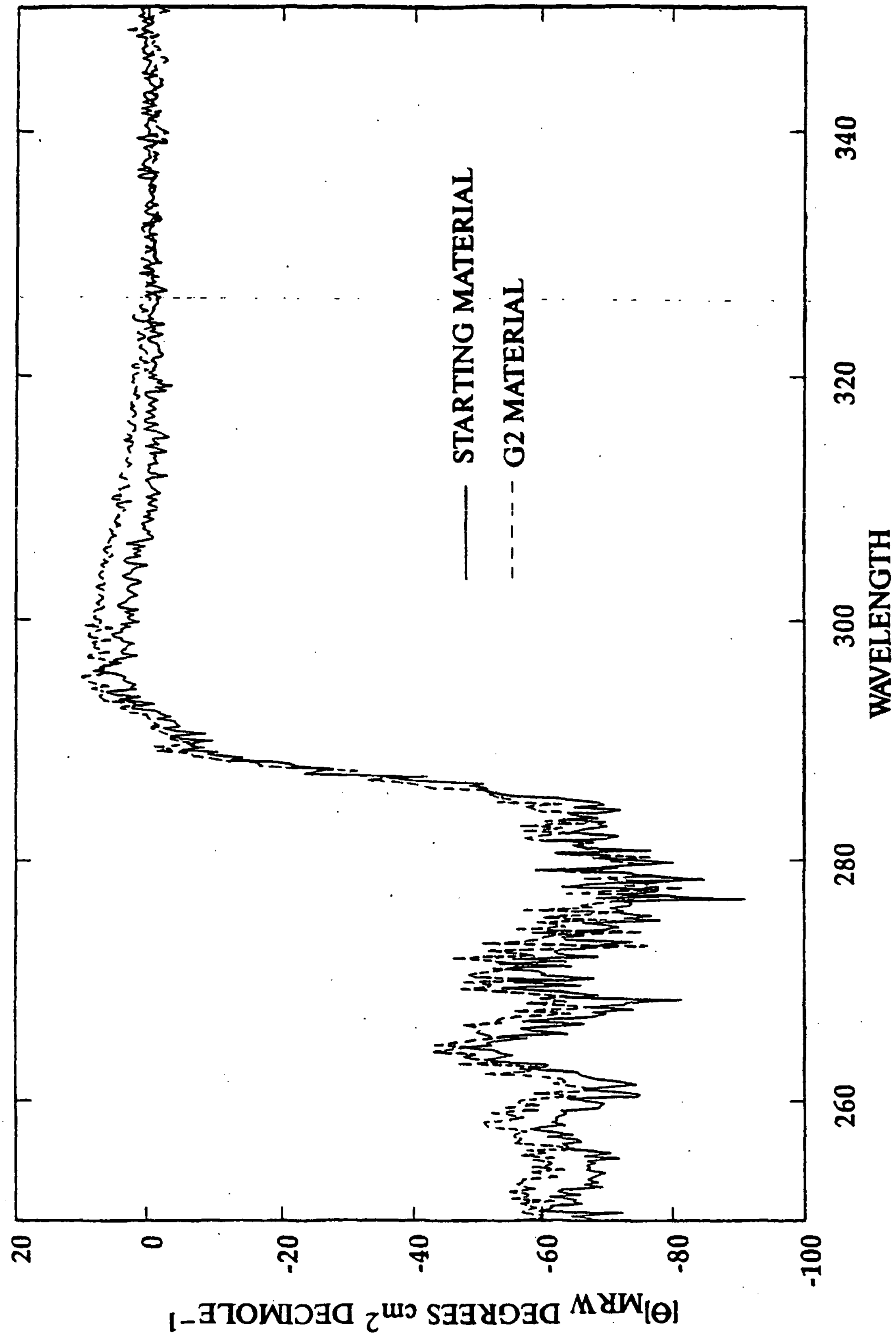
STARTING MATERIAL
G2 MATERIAL
20
0
-20
-40
-60
-80
-100
[Θ]MRW DEGREES cm2 DECIMOLE-1
260
280
300
320
340
WAVELENGTH

FIG. 10B

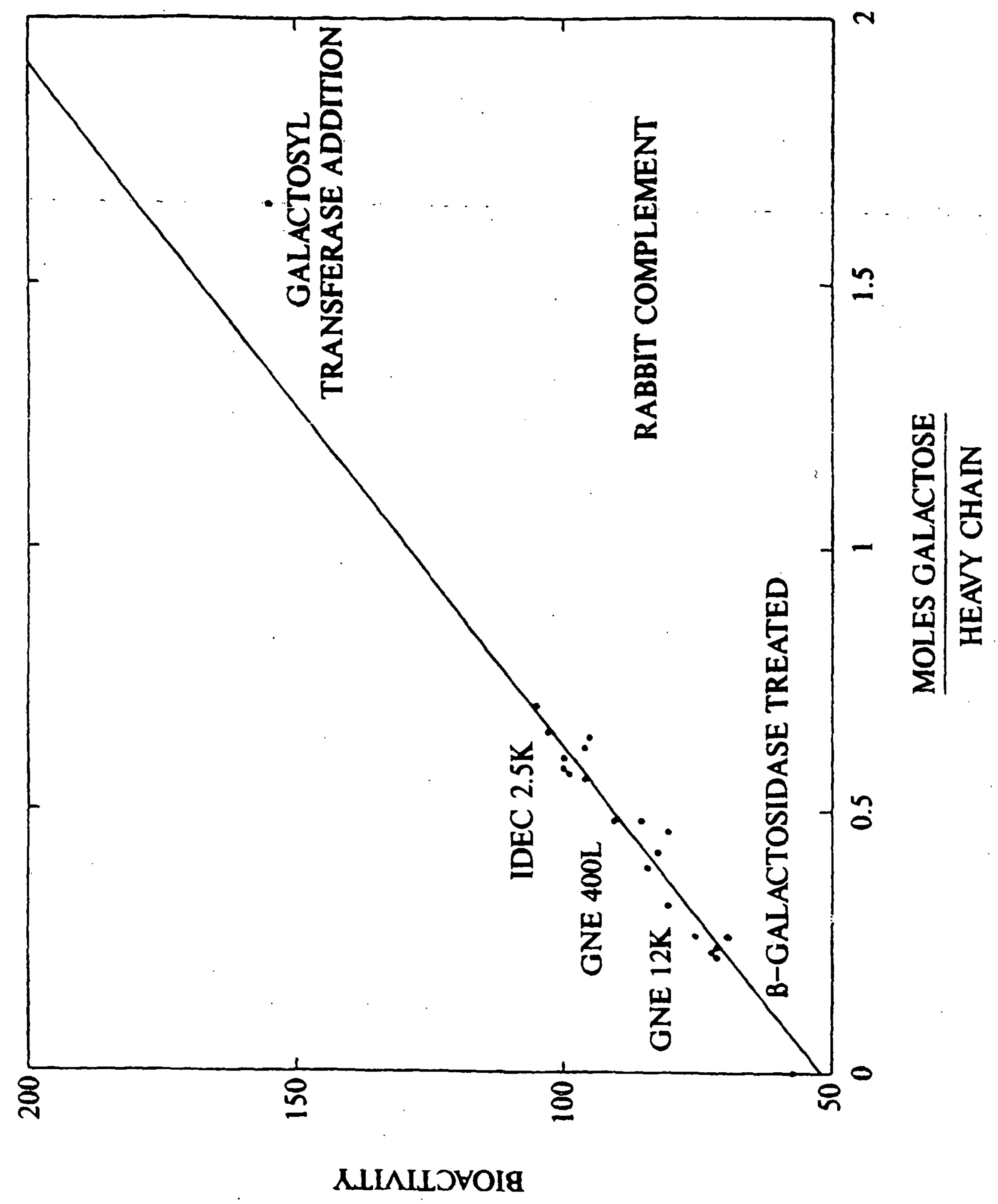
GALACTOSYL
TRANSFERASE ADDITION
RABBIT COMPLEMENT
IDEC 2.5K
GNE 400L
GNE 12K
B-GALACTOSIDASE TREATED
200
150
100
50
BIOACTIVITY
0
0.5
1
1.5
2
MOLES GALACTOSE
HEAVY CHAIN

FIG. 11

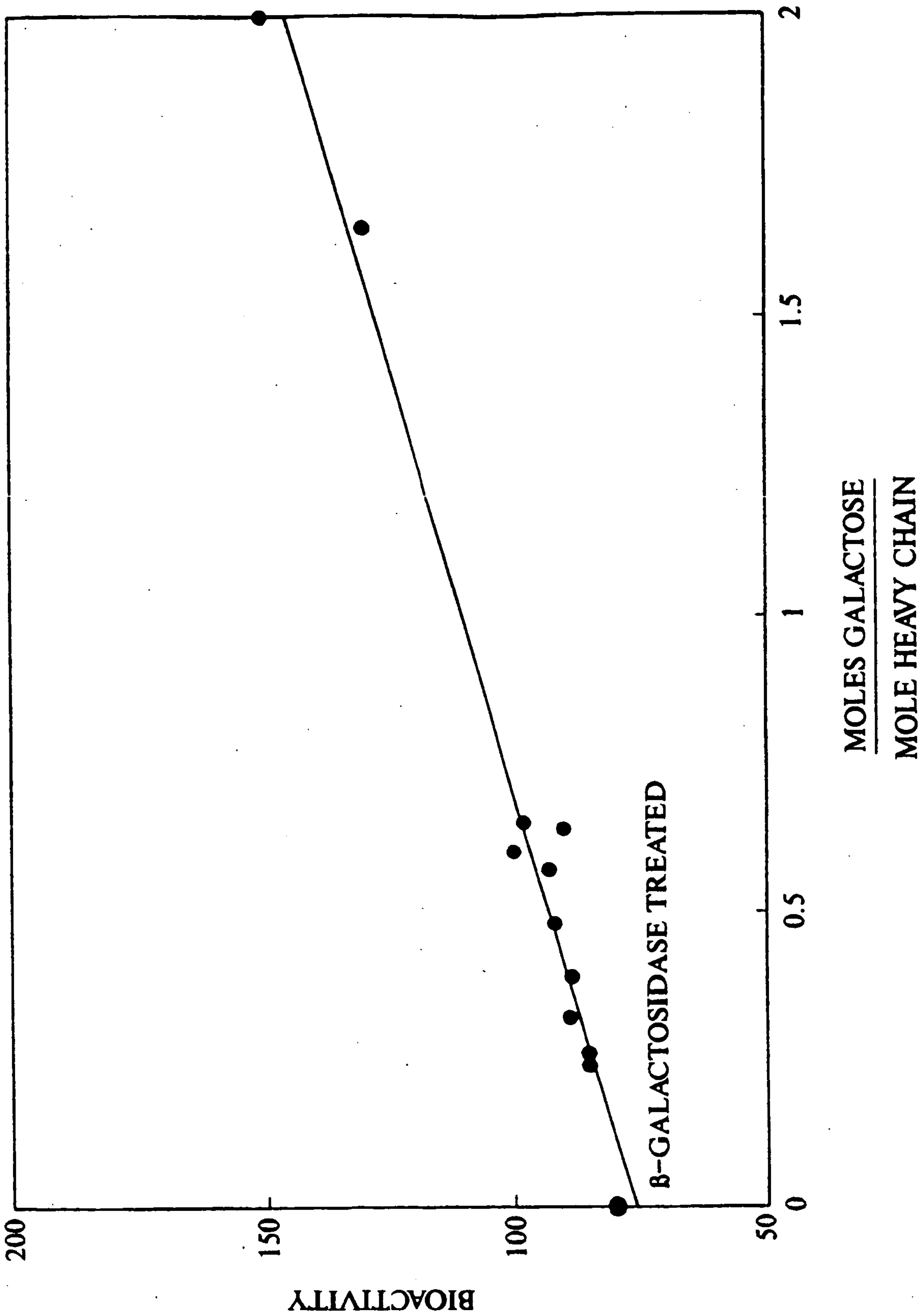

BIOACTIVITY
200
150
100
50
0
0.5
1
1.5
2
B-GALACTOSIDASE TREATED
MOLES GALACTOSE
MOLE HEAVY CHAIN


FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5116964 A
- US 5565335 A
- US 5610297 A
- WO 9523865 A
- WO 9519181 A
- WO 9220798 A
- WO 9630046 A
- US 5096816 A
- US 5047335 A
- US 5510261 A
- US 5278299 A
- WO 9411026 A
- US 4816567 A, Cabilly
- WO 8902922 A
- EP 314317 A
- WO 9117769 A
- WO 9222653 A
- US 4745055 A
- EP 256654 A
- EP 120694 A
- EP 125023 A
- EP 255694 A
- EP 266663 A
- WO 8803559 A
- EP 307247 A
- EP 669836 B


### Non-patent literature cited in the description


- **ASHKENAZI et al.** *Proc. Natl. Acad. Sci. USA,* 1991, vol. 88, 1-5351053
- **ABRAHAM et al.** *Sec. Intern. Autumnal Them. Meeting on Sepsis,* 1995
- **ST JOHN et al.** *Chest,* 1993, vol. 103, 932
- **FILCHER et al.** *Blood,* vol. 77, 249-256
- **STEPPE et al.** *Transplant Intl.,* 1991, vol. 4, 3-7
- **HOURMANT et al.** *Transplantation,* 1994, vol. 58, 377-380
- **PRESTA et al.** *J. Immunol.,* 1993, vol. 151, 2623-2632
- **CARTER et al.** *Proc. Natl. Acad. Sci. USA,* 1992, vol. 89, 4285-4289
- **JIN KIM et al.** *Growth Factors,* 1992, vol. 7, 53-64
- **MALONEY et al.** *Blood,* 1994, vol. 84, 2457-2466
- **LIU et al.** *J. Immunol.,* 1987, vol. 130, 3521-3526
- **JEFFERIS ; LUND.** *Chem. Immunol.,* 1997, vol. 65, 111-128
- **WRIGHT ; MORRISON.** *TibTECH,* 1997, vol. 15, 26-32
- **BOYD et al.** *Mol. Immunol.,* 1996, vol. 32, 1311-1318
- **WITTWER A. ; HOWARD, S.C.** *Biochem.,* 1990, vol. 29, 4175-4180
- **WYSS ; WAGNER.** *Curr. Opin. Biotech.,* 1996, vol. 7, 409-416
- **HART.** *Curr. Opin. Cell Biol.,* 1992, vol. 4, 1017-1023
- **GOOCHEE et al.** *Bio/Technology,* 1991, vol. 9, 1347-1355
- **PAREKH, R.B.** *Curr. Opin. Struct. Biol.,* 1991, vol. 1, 750-754
- **MALHOTRA et al.** *Nature Med.,* 1995, vol. 1, 237-243
- **TSUCHIYA et al.** *J. Rheum.,* 1989, vol. 16, 285-290
- **SHEELEY et al.** *Analytical Biochem.,* 1997, vol. 247, 102-110
- **PAREKH et al.** *Biochemistry,* 1989, vol. 28, 7644-7662
- **HSE, T.A. et al.** *J. BioL Chem.,* 1997, vol. 272, 9062-9070
- **WERNER, R. ; NOE, W.** *Drug Res.,* 1993, vol. 43, 1134-1249
- **HAYTER et al.** *Biotech, and Bioeng.,* 1992, vol. 39, 327-335
- **BORYS et al.** *Biotech and Bioeng.,* 1994, vol. 43, 505-514
- **BORYS et al.** *Bio/technology,* 1993, vol. 11, 720-724
- **HEARING et al.** *J. Cell Biol.,* 1989, vol. 108, 339-353
- **GOOCHEE et al.** Frontiers in Bioprocessing II. American Chemical Society, 1992, 199-240
- **CHOTIGEAT, W.** *Cytotech.,* 1994, vol. 15, 217-221
- **PARK et al.** *Biotech. Bioeng.,* 1992, vol. 40, 686-696

- **COX ; MCCLURE.** *In Vitro,* 1983, vol. 19, 1-6
- **MIZUTANI et al.** *Biochem. Biophys. Res. Comm.,* 1992, vol. 187, 664-669
- **LE GROS et al.** *Lymph. Res.,* 1985, vol. 4 (3), 221-227
- **KLAMA K et al.** The abnormalities in the glycosylation of IgG. *REUMATOLOGIA,* 1996, vol. 34 (1), 26-30
- **WORMALD et al.** *Biochemistry,* 1997, vol. 36, 1370-1380
- **WEITZHANDLER et al.** *J. Pharm. Sci.,* 1994, vol. 83, 1760
- **REFF et al.** *Blood,* 1994, vol. 83, 435-445
- **PROVENCER ; GLOCKNER.** *Biochem.,* 1981, vol. 20, 33-37
- **HUBBARD ; IVATT.** *Ann., Rev. Biochem.,* 1981, vol. 50, 555-583
- *J. Biol. Chem,* 1982, vol. 257, 3347
- *J. Biol. Chem.,* 1982, vol. 257, 3352
- **KABAT E.A.** *Adv. Protein Chem.,* 1978, vol. 32, 1-75
- **EDELMAN, G.M. et al.** *Proc. Natl. Acad. Sci. USA,* 1969, vol. 63, 78-85
- **ELLISON et al.** *EMBO J.,* 1982, vol. 1, 403-407
- **BEALE ; FEINSTEIN.** *Q. Rev. Biophys.,* 1976, vol. 9, 253-259
- **JEFFERIS et al.** *Immunol. Letts.,* 1995, vol. 44, 111-117
- **KABAT et al.** Sequences of proteins of immunological interest. NIH Publication No. 91-3242
- **PRESS et al.** *NEJM,* 1993, vol. 329, 12219-12223
- **KAMINSKI et al.** *NEJM,* 1993, 329
- **MCLAUGHLIN et al.** *Proc. Am. Soc. Clin. Oncol.,* 1996, vol. 15, 417
- **LIU et al.** *J. Immunol.,* 1987, vol. 139, 3521
- **MALONEY et al.** *Blood,* 1994, vol. 84, 2457
- **KOHLER et al.** *Nature,* 1975, vol. 256, 495
- **MAGE ; LAMOYI.** Monoclonal_Antibody Production Techniques and Applications. Marcel Dekker, Inc, 1987, 79-97
- **MORRISON et al.** *Proc. Natl. Acad. Sci. U.S.A.,* 1984, vol. 81, 6851
- **JONES et al.** *Nature,* 1986, vol. 321, 522
- **REICHMANN et al.** *Nature,* 1988, vol. 332, 323
- **PRESTA.** *Curr. Op. Struct. Biol.,* 1992, vol. 2, 593
- **CAPON et al.** *Nature,* 1989, vol. 337, 525-531
- **TRAUNECKER et al.** *Nature,* 1989, vol. 339, 68-70
- **BYM et al.** *Nature,* 1990, vol. 344, 667-670
- **WATSON et al.** *J. Cell. Biol.,* 1990, vol. 110, 2221-2229
- **WATSON et al.** *Nature,* 1991, vol. 349, 164-167
- **ARUFFO et al.** *Cell,* 1990, vol. 61, 1303-1313
- **LINSLEY et al.** *J. Exp. Med.,* 1991, vol. 173, 721-730
- **LISLEY et al.** *J. Exp. Med.,* vol. 174, 561-569
- **STAMENKOVIC et al.** *Cell,* vol. 66, 1133-1144
- **ASHKENAZI et al.** *Proc. Natl. Acad. Sci. USA,* 1991, vol. 88, 10535-10539
- **LESSLAUER et al.** *Eur. J. Immunol.,* 1991, vol. 27, 2883-2886
- **PEPPEL et al.** *J. Exp. Med.,* 1991, vol. 174, 1483-1489
- **BENNETT et al.** *J. Biol. Chem.,* 1991, vol. 266, 23060-23067
- **KURSCHNER et al.** *J. Biol. Chem.,* 1992, vol. 267, 9354-9360
- **CHALUPNY et al.** *PNAS USA,* 1992, vol. 89, 10360-10364
- **ASHKENAZI et al.** *Intem. Rev. Immunol.,* 1991, vol. 10, 219-227
- **ASHKENAZI, A. et al.** *PNAS USA,* 1991, vol. 88, 10535-10539
- **DIETSCH et al.** *J. Immunol. Methods,* 1993, vol. 162, 123
- **KOHLER ; MILSTEIN.** *Nature,* 1975, vol. 256, 495
- **GODING.** Monoclonal Antibodies: Principles and Practice. Academic Press, 1986, 59-103
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **BOERNER et al.** *J. Immunol.,* 1991, vol. 147 (1), 86-95
- **MUNSON ; POLLARD.** *Anal. Biochem.,* 1980, vol. 107, 220
- **GODING.** Monoclonal Antibodies: Principles and Practice. Academic Press, 1986, 59-104
- **JAKOBOVITS et al.** *Proc. Natl. Acad. Sci. USA,* 1993, vol. 90, 2551-255
- **JAKOBOVITS et al.** *Nature,* 1993, vol. 362, 255-258
- **MCCAFFERTY et al.** *Nature,* 1990, vol. 348, 552-554
- **CLACKSON et al.** *Nature,* 1991, vol. 352, 624-628
- **MARKS et al.** *J. Mol. Biol.,* 1991, vol. 22, 581-597
- **MARK et al.** *Bio/Technol.,* 1992, vol. 10, 779-783
- **WATERHOUSE et al.** *Nuc. Acids Res.,* 1993, vol. 21, 2265-2266
- **JONES et al.** *Nature,* 1986, vol. 321, 522-525
- **RIECHMANN et al.** *Nature,* 1988, vol. 332, 323-327

- **VERHOEYEN et al.** *Science,* 1986, vol. 239, 1534-1536
- **FAULKNER et al.** *Nature,* 1982, vol. 298, 286
- **MORRISON.** *J. Immun.,* 1979, vol. 123, 793
- **KÖHLER et al.** *Proc. Natl. Acad. Sci. USA,* 1980, vol. 77, 2197
- **RASO et al.** *Cancer Res.,* 1981, vol. 41, 2073
- **MORRISON et al.** *Ann. Rev. Immunol.,* 1984, vol. 2, 239
- **MORRISON.** *Science,* 1985, vol. 229, 1202
- **MORRISON et al.** *Proc. Natl. Acad. Sci. USA,* 1984, vol. 81, 6851
- **ZETTMEISSL et al.** *DNA Cell Biol. USA,* vol. 9, 347-353
- **BYRN et al.** *Nature,* 1990, vol. 344, 667-670
- **LISLEY et al.** *J. Exp. Med.,* 1991, vol. 174, 561-569
- **STAMENKOVIC et al.** *Cell,* 1991, vol. 66, 1133-1144
- **RIDGWAY ; GORMAN.** *J. Cell. Biol.,* 1991, vol. 115
- **HOOGENBOOM et al.** *Mol. Immunol,* 1991, vol. 28, 1027-1037
- **CHO ; URLAUB ; CHASIN.** *Proc. Natl. Acad. Sci. USA,* 1980, vol. 77, 4216
- **GRAHAM et al.** *J. Gen Virol.,* 1977, vol. 36, 59
- **TM4, MATHER.** *Biol. Reprod.,* 1980, vol. 23, 243-251
- **MATHER et al.** *Annals N.Y. Acad. Sci.,* 1982, vol. 383, 44-68
- *J. Immunol. Methods,* 1983, vol. 56, 221-234
- Animal Cell Culture: A Practical Approach. Oxford University Press, 1992
- **BOEGGEMAN et al.** *Prot. Eng.,* 1993, vol. 6 (7), 779-785
- **SCHWEINTECK.** *Gene,* 1994, vol. 145 (2), 299-303
- **KLEENE et al.** *Biochem. Biophys. Res. Commun.,* 1994, vol. 201 (1), 160-167
- **CHATTERJEE et al.** *Int. J. Biochem Cell. Biol.,* 1995, vol. 27 (3), 329-336
- **HERRMANN et al.** *Protein. Expr. Purif.,* 1995, vol. 6 (1), 72-78
- **ANUMULA et al.** *Anal. Biochem.,* 1991, vol. 195, 269-280
- **YAO et al.** *Anal Biochem.,* 1989, vol. 179, 332-335
- **WARREN, L.** *J. Biol Chem,* 1959, vol. 238 (8
- **HASELBECK et al.** *Glycoconjugate J.,* 1990, vol. 7, 63
- **WORALD et al.** *Biochem.,* 1997, vol. 36, 1370-1380
- **SHEELEY et al.** *Anal. Biochem.,* 1997, vol. 247, 102-110
- **CANT et al.** *Cytotechnology,* 1994, vol. 15, 223-228
- **IWASE et al.** *J. Biochem.,* 1996, vol. 120, 393-397
- **SHEELEY et al.** *Analytical Biochemistry,* 1997, vol. 247, 102-110
- **GAZZANO-SANTORO et al.** *J. Immunol. Methods,* 1997, vol. 202, 163-171
- Remington's Pharmaceutical Sciences. 1980
- Chemotherapy Service. Williams & Wilkins, 1992
- **CARTER et al.** *Proc. natl. Acad. Sci. USA,* 1992, vol. 89, 4285
- **KIM et al.** *Growth Factors,* 1992, vol. 7, 53-64
- **PRESTA et al.** *J. Immunol.,* 1993, vol. 151, 2623
- **ASHKENAZI et al.** *Proc. Natl. Acad. Sci. USA,* 1991, vol. 88, 10535
- **REFF et al.** *Blood,* 1994, vol. 83, 435-45
- **GAZZANO-SANTORO.** *J. Immunol. Meth.,* 1997, vol. 202, 163-171
- **GLOCKNER.** *Biochem.,* 1981, vol. 20, 33-37
- **PROVENCER.** *Comput. Phys. Commun.,* 1982, vol. 27, 229-242